# EUROPEAN PATENT APPLICATION

(11) **EP 4 560 003 A1**
(43) Date of publication of application: **28.05.2025**
(21) Application number: 24817463.3
(22) Date of filing: 19.07.2024
(51) Int. Cl.: C12M 1/00, C12Q 1/6874, C12Q 1/6869, G03F 7/00

(54) **CHIP AND PREPARATION METHOD THEREFOR**

(30) Priority: 20.06.2023 CN 202310740588
(71) Applicant: GeneMind Biosciences Company Limited, Shenzhen City, 518023 Guangdong (CN); Nanjing Genemind Biosciences Company Limited, Nanjing, Jiangsu 210000 (CN)
(72) Inventor: GU, Jiaqiang, Shenzhen, Guangdong 518023 (CN); LIN, Zhifeng, Shenzhen, Guangdong 518023 (CN); CENG, Dezhi, Shenzhen, Guangdong 518023 (CN); YANG, Xinlin, Shenzhen, Guangdong 518023 (CN); ZHOU, Lifang, Shenzhen, Guangdong 518023 (CN); LI, Guang, Shenzhen, Guangdong 518023 (CN); CHEN, Fang, Shenzhen, Guangdong 518023 (CN)
(74) Representative: Schiweck Weinzierl Koch Patentanwälte Partnerschaft mbB
(86) International application number: PCT/CN2024/106451
(87) International publication number: WO 2024/260470

(57) **Abstract**

The present application discloses a chip, comprising a chip substrate provided with regularly arranged geometric structures on at least one surface. The geometric structure has a height of 10 nm to 100 µm in a direction perpendicular to the surface of the chip and a maximum dimension of 10 nm to 100 µm in a direction parallel to the surface of the chip. The distance between adjacent geometric structures is 10 nm to 100 µm. By forming the regularly arranged geometric structures on the chip substrate, the present application allows a controllable arrangement of biomolecule detection sites and a high density, and can thus improve the detection throughput and detection cost-efficiency. The dimensional parameters of the biochip geometric structure provided herein can improve the sequencing quality, and feature high repeatability, good stability, and resistance to multiple cycles of reagent scouring.

## Description

### TECHNICAL FIELD

The present disclosure relates to the field of biotechnology, and in particular to, a chip and a method for preparing same.

### BACKGROUND

Chips for biomolecule detecting platforms are based on the principle of specific interaction between biomolecules and integrate biochemical analysis processes on the chip surface to achieve high-throughput, fast detection of DNA, RNA, polypeptide, protein, and other biological components. Biochips possess the advantages of high throughput, miniaturization, automation, and the like. They are widely used in the fields of gene sequencing, protein detection, small biomolecule (such as saccharides) detection, and the like, and provide feasible technical schemes for the techniques of nucleic acid sequence analysis, drug screening, and large-scale biological sample detection.

Biomolecule detection sites can be formed on the chip surface through geometric structures with specific shapes. A patterned arrangement of the sites may facilitate the control of the arrangement density of the geometric structures on the chip surface and can thereby achieve the simultaneous detection of various samples, thus improving the detection throughput. Configuring the chip surface as a patterned surface with a regular arrangement of geometric structures is beneficial to improving the arrangement density of the chips and improving the surface utilization rate.

The detection of nucleic acid sequence on chip surface features a broad application range. Studies have shown that the structural design of chips, as a medium and a reactor of sequencing, will influence various parameters and indicators reflecting the sequencing results, thus affecting the accuracy of the sequencing results. How to design a proper chip micro/nano structure to improve the sequencing stability and accuracy upon use of the chip in sequencing, has become a concern of those skilled in the art.

### SUMMARY

In various studies, the inventor found that in a chip-based method for nucleic acid sequencing, the dimensions of the geometric structure on the chip surface, including the height of the geometric structure in the vertical direction, the dimension of the geometric structure in the horizontal direction, and the depth of the geometric structure, may influence the stability and the accuracy of the sequencing results. Such an effect is reflected by the sequencing indicators or parameters obtained during the sequencing process, for example: the stability of clonal clusters obtained after the amplification of nucleic acid molecules within the geometric structure, the intensity of signals captured during the sequencing, parameters such as Q30, resolution of multiple tags on a sample and the match rate with reference genomes in sequencing results indicating the quality of sequences of interest (reads), etc.

In view of this, the present disclosure provides a chip and a method for preparing same advantageous to improving the sequencing quality by designing the dimensions of the geometric structure on the chip.

The first aspect of the present disclosure provides a patterned chip, including a chip substrate provided with regularly arranged geometric structures on at least one surface. The geometric structure has a height of 10 nm to 100 µm in a direction perpendicular to the surface of the chip and a maximum dimension of 10 nm to 100 µm in a direction parallel to the surface of the chip. The distance between adjacent geometric structures is 10 nm to 100 µm.

By forming the regularly arranged geometric structures on the substrate, the present disclosure can improve the stability of sequencing and the accuracy of sequencing results. Moreover, the regularly arranged geometric structures can improve the arrangement density of the geometric structures on the chip and thus the detection throughput; the detection sites corresponding to the geometric structures can be positioned through the regularly arranged geometric structures, thereby facilitating the synchronous detection of multiple nucleic acid molecules on the surface of the same chip and improving the detection cost-efficiency.

As one possible embodiment, the geometric structure has a height of 100 nm to 1 µm in a direction perpendicular to the surface and a maximum dimension of 100 nm to 1 µm in a direction parallel to the surface;
the distance between adjacent geometric structures is 100 nm to 1 µm.

As one possible embodiment, the geometric structure has a height of 100 nm to 1 µm in a direction perpendicular to the surface of the chip and a maximum dimension of 100 nm to 1 µm in a direction parallel to the surface of the chip;
the distance between adjacent geometric structures is 100 nm to 1 µm.

As one possible embodiment, the geometric structure is a depression or protrusion formed on the surface of the chip substrate.

As one possible embodiment, the geometric structure is one or a combination of more of a cylinder, a cone, a conical frustum, a prism, a pyramid, a frustum, or an irregular shape.

As one possible embodiment, the surface is provided with at least one array unit having the regularly arranged geometric structures.

As one possible embodiment, the array unit is one or more of a square array unit, a rectangular array unit, and a honeycomb array unit.

As one possible embodiment, the chip further includes a first cover plate bonded to the surface of the chip substrate provided with the geometric structure, and the first cover plate is made of a material having a light transmittance of 95% or greater.

As one possible embodiment, the surface of the first cover plate facing the chip substrate is a flat surface.

As one possible embodiment, the surface of the first cover plate facing the chip substrate is provided with regularly arranged geometric structures, and the geometric structure of the first cover plate is identical to or different from that of the chip substrate.

As one possible embodiment, the chip substrate is provided with regularly arranged geometric structures on the two surfaces.

As one possible embodiment, the chip includes a first cover plate and a second cover plate respectively bonded to the two surfaces of the chip substrate, and the first cover plate and the second cover plate are made of a material having a light transmittance of 95% or greater.

As one possible embodiment, two surfaces of the first cover plate and the second cover plate are flat surfaces.

As one possible embodiment, the first cover plate is provided with a geometric structure on one surface, the geometric structure of the first cover plate is arranged opposite to the geometric structure of the chip substrate, and two surfaces of the second cover plate are flat surfaces.

As one possible embodiment, the first cover plate and the second cover plate are both provided with a geometric structure on one surface, and the geometric structures on the first cover plate and the second cover plate are respectively opposite to the geometric structures on the two surfaces of the chip substrate.

As one possible embodiment, the chip includes a polymer layer arranged on the surface provided with the geometric structure.

As one possible embodiment, the polymer layer is formed from a polymer containing a first functional group selected from one or more of sulfydryl, formyl, hydroxyl, carboxyl, amino, alkenyl, alkynyl, and azido.

As one possible embodiment, the polymer layer is bonded to the surface of the chip substrate and/or the cover plate via a coupling molecule.

As one possible embodiment, the coupling molecule has the following formula: XₐSi(Y_{b})M_{c}Z, where
X is H, F, Cl, Br, I, C₁₋₆ alkyl, C₁₋₆ haloalkyl, or C₁₋₆ alkoxy;
Y is selected from C₁₋₁₀ alkyl;
M_{c} is selected from C₁₋₅₀ alkylene, -(OCH₂CH₂)_{c}-, and -(CH(OH)CH₂-)_{c};
Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl;
a, b, and c are integers meeting the following conditions: 0 ≤ a ≤ 3, 0 ≤ b ≤ 3, and a + b = 3; 1 ≤ c ≤ 50.

As one possible embodiment, the chip further includes a first film layer arranged between the chip substrate and/or the cover plate and the polymer layer, where the first film layer is made of a material different from that of the surface to which it is bonded.

As one possible embodiment, the first film layer is selected from one or more of a metal film, a metal oxide film, and an inorganic oxide film.

As one possible embodiment, the first film layer is made of at least one of Au, Al, and SiO₂.

As one possible embodiment, the first film layer has a thickness of 10 to 1000 nm.

As one possible embodiment, a glue layer is arranged between the chip substrate and the cover plate, and the glue layer has a channel arranged in a length direction of the chip; the geometric structure is arranged on the surface of the chip substrate and/or the cover plate in a region where the channel is located, and the shapes and/or arrangements of the geometric structures in different channels on the same surface are identical or different.

The second aspect of the present disclosure provides a method for preparing a chip, including:
a) forming regularly arranged geometric structures on at least one surface of a substrate,

where the geometric structure has a height of 10 nm to 100 µm in a direction perpendicular to the surface of the substrate and a maximum dimension of 10 nm to 100 µm in a direction parallel to the surface of the substrate;
the distance between adjacent geometric structures is 10 nm to 100 µm.

As one possible embodiment, the substrate is a chip substrate and/or a cover plate.

As one possible embodiment, the method further includes:
b) forming a polymer layer on the geometric structure.

As one possible embodiment, the geometric structure is a depression formed on the surface of the chip substrate and/or the cover plate, and b) includes:
bonding a polymer containing a first functional group to the surface provided with the geometric structure; and
polishing the polymer to remove the polymer outside the region where the geometric structure is located, so as to form the polymer layer on the geometric structure.

As one possible embodiment, the polishing is chemical polishing and/or mechanical polishing.

As one possible embodiment, the geometric structure is a protrusion formed on the surface of the chip substrate and/or the cover plate, and the method includes, before a):
bonding the polymer containing the first functional group to the surface of the substrate.

As one possible embodiment, the first functional group is selected from one or more of sulfydryl, formyl, hydroxyl, carboxyl, amino, alkenyl, alkynyl, and azido.

As one possible embodiment, the method further includes, before bonding the polymer: processing the substrate with a coupling molecule.

As one possible embodiment, the coupling molecule has the following formula: XₐSi(Y_{b})M_{c}Z, where
X is H, F, Cl, Br, I, C₁₋₆ alkyl, C₁₋₆ haloalkyl, or C₁₋₆ alkoxy;
Y is selected from C₁₋₁₀ alkyl;
M_{c} is selected from C₁₋₅₀ alkylene, -(OCH₂CH₂)_{c}-, and -(CH(OH)CH₂-)_{c};
Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl;
a, b, and c are integers meeting the following conditions: 0 ≤ a ≤ 3, 0 ≤ b ≤ 3, and a + b = 3; 1 ≤ c ≤ 50.

As one possible embodiment, the method further includes: before a), or after a) and before b), preparing a first film layer on the surface provided with the geometric structure, where the first film layer and the substrate are made of different materials.

As one possible embodiment, the first film layer is selected from one or more of a metal film, a metal oxide film, and an inorganic oxide film.

As one possible embodiment, the first film layer is made of at least one of Au, Al, and SiO₂.

As one possible embodiment, the first film layer has a thickness of 10 to 1000 nm.

As one possible embodiment, the substrate includes at least a chip substrate, and the method further includes:
bonding the cover plate to the surface of the chip substrate provided with the geometric structure using an adhesive material, and forming a channel in the adhesive material in a length direction of the chip.

As one possible embodiment, the chip is the chip according to the first aspect.

### BRIEF DESCRIPTION OF THE DRAWINGS

In order to more clearly illustrate the technical schemes in the examples of the present application or the prior art, the drawings required for use in the description of the examples or the prior art will be briefly described below. It is obvious that the drawings in the description below are only some examples of the present application, and other drawings can be derived from these drawings by those of ordinary skill in the art without creative efforts.

Among the drawings:
FIG. 1 is a schematic cross-sectional view of a chip substrate with cylindrical depression geometric structures according to one example of the present application;
FIG. 2 is a schematic top view of a chip substrate with cylindrical depression geometric structures according to one example of the present application;
FIG. 3 is a cross-sectional view of a chip substrate with cylindrical depression geometric structures according to one example of the present application;
FIG. 4 is a top view of a chip substrate with cylindrical depression geometric structures according to one example of the present application;
FIG. 5 is a schematic cross-sectional view of a chip substrate with cylindrical protrusion geometric structures according to one example of the present application;
FIG. 6 is a schematic top view of a chip substrate with cylindrical protrusion geometric structures according to one example of the present application;
FIG. 7 is a cross-sectional view of a chip substrate with cylindrical protrusion geometric structures according to one example of the present application;
FIG. 8 is a structural schematic of a chip having a chip substrate with the geometric structure on one surface according to one example of the present application;
FIG. 9 is a structural schematic of another chip having a chip substrate with the geometric structure on one surface according to one example of the present application;
FIG. 10 is a structural schematic of a chip having a chip substrate with the geometric structure on two surfaces according to one example of the present application;
FIG. 11 is a structural schematic of another chip having a chip substrate with the geometric structure on two surfaces according to one example of the present application;
FIG. 12 is a structural schematic of yet another chip having a chip substrate with the geometric structure on two surfaces according to one example of the present application;
FIG. 13 is a schematic flow chart of the method for preparing a biochip according to Example 1 of the present application;
FIG. 14 is a top view of the biochip provided in Example 1 of the present application;
FIG. 15 is a cross-sectional view of the biochip provided in Example 1 of the present application;
FIG. 16 is a statistical diagram of fluorescence intensities of nucleic acid amplifications on biochips of different well depths provided in Examples 1-4 of the present application;
FIG. 17 is a statistical diagram of amplification occupancy, Q30, resolution, and match rate of sequencing amplifications on biochips of different well depths provided in Examples 1-4 of the present application;
FIG. 18 is a schematic flow chart for preparing the biochip provided in Example 5 of the present application;
FIG. 19a is a structural schematic of the biochip prepared in Comparative Example 1;
FIG. 19b is a fluorescent image illustrating the multiple channels of the biochip prepared in Comparative Example 1;
FIG. 19c is a partial surface fluorescent image of the biochip prepared in Comparative Example 1;
FIG. 19d is a structural schematic of the biochip prepared in Example 5;
FIG. 19e is a fluorescent image illustrating the multiple channels of the biochip prepared in Example 5;
FIG. 19f is a partial surface fluorescent image of the biochip prepared in Example 5;
FIG. 20 is a schematic of the method for preparing the biochip provided in Example 6 of the present application;
FIG. 21a is an image illustrating the surface of the biochip prepared in Comparative Example 2;
FIG. 21b is a partially enlarged view of FIG. 21a;
FIG. 21c is an image illustrating the surface of the biochip prepared in Example 6;
FIG. 21d is an enlarged view illustrating the fluorescence on the surface of the biochip prepared in Example 6;
FIG. 22 is a schematic flow chart for preparing the biochip provided in Example 7 of the present application;
FIG. 23a is an image illustrating the surface of the biochip prepared in Comparative Example 3;
FIG. 23b is a fluorescent image illustrating the multiple channels of the biochip prepared in Comparative Example 3;
FIG. 23c is an enlarged view illustrating the fluorescence on the surface of the biochip prepared in Comparative Example 3;
FIG. 23d is an image illustrating the surface of the biochip prepared in Example 5; and
FIG. 23e is an enlarged view illustrating the fluorescence on the surface of the biochip prepared in Example 5.

### DETAILED DESCRIPTION

The technical schemes in the examples of the present application will be described below clearly and comprehensively in conjunction with the drawings in the examples of the present application.

It is obvious that the described examples are part of the examples of the present application, but not all of them. On the basis of the examples of the present application, all other examples obtained by those of ordinary skill in the art without creative efforts shall fall within the protection scope of the present application.

The present disclosure provides a patterned biochip and a method for preparing same, which may be implemented by those skilled in the art with appropriately improved process parameters with reference to the present disclosure. It is specifically noted that all such substitutions and modifications will be apparent to those skilled in the art and are included in the present disclosure. Although the method and use of the present disclosure have been described in terms of preferred examples, it will be apparent that those skilled in the art can make modifications or proper changes and combinations to the method and use disclosed herein to implement and apply the techniques of the present disclosure without departing from the content, spirit, and scope of the present disclosure.

The examples of the present application provide a chip, including a chip substrate configured for carrying a nucleic acid molecule of interest and providing a reaction site or a detection site for the nucleic acid molecule. In the examples of the present application, the chip substrate is provided with a geometric structure on at least one surface, and thus, the chip may be referred to as a patterned chip. A flow channel including a reaction region can be formed after the chip is encapsulated, and therefore, the chip provided with the geometric structure is also referred to as a flow cell.

In the examples of the present application, the chip substrate is stable and chemically inert, such that when the nucleic acid molecules are immobilized on the surface of the chip substrate, the material of the chip substrate dose not change the physicochemical properties and the structure of the nucleic acid molecules including, but not limited to, the composition of the nucleic acid molecules, and the spatial conformation, the two-dimensional structure and even the three-dimensional structure of the nucleic acid molecules. In some specific embodiments, the material of the chip substrate includes but is not limited to: glass; quartz; a silicon-based material such as silicon dioxide, polysilicon, single-crystal silicon, etc.; a polymer such as hexamethyldisilazane (HMDS), polyethylene (PE), polypropylene (PP), etc.; a metal, e.g., aluminum, etc. The material may be one of the above, or may be a composite substrate material containing a combination of two or more. It will be appreciated that when the chip substrate consists of a plurality of materials, the composition may include at least the following two cases. In one embodiment, two or more materials are mixed to give a mixed material, which is used as a starting material to prepare the chip substrate. At this point, the chip substrate is present as one layer of material. Preferably, similar materials may be selected for mixing to prepare the chip substrate in consideration of the processability of the chip substrate and the performance of the resultant product. Illustratively, a mixture containing silicon dioxide and polysilicon is used to prepare the chip substrate, or a mixture containing polyethylene (PE) and polypropylene (PP) is used to prepare the chip substrate, etc. In another embodiment, two or more different materials are selected to prepare a composite layer having at least two layer structures as the chip substrate. Each layer structure may be made of one material, or may be made of a composite of two or more different materials according to the above embodiment. The layer structures may be arranged in a certain structure, such as a laminated structure, etc.

The dimension of the substrate is not particularly limited and can be designed as needed. In some examples, for convenience of processing, regularly arranged geometric structures are formed on a wafer with a large area, and the wafer is then cut to give chip substrates with a proper size. The size of the wafer is not limited, and may be 6 inches, 8 inches, 12 inches, etc.

The chip substrate provided in the examples of the present application is provided with a geometric structure. The geometric structure includes, but is not limited to, a cylinder, a cone, a conical frustum, a prism, a pyramid, a frustum, or an irregular shape, or may also be a combination of two of a cylinder, a cone, a conical frustum, a prism, a pyramid, a frustum, or an irregular shape.

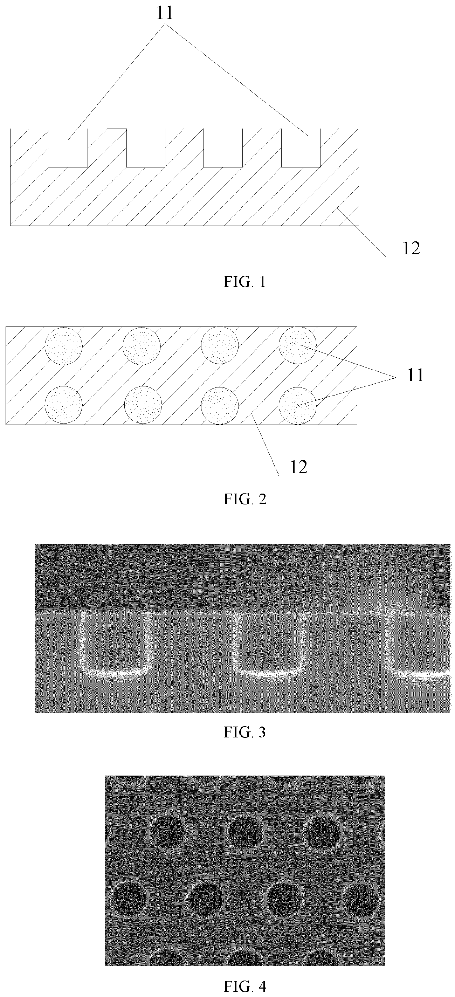

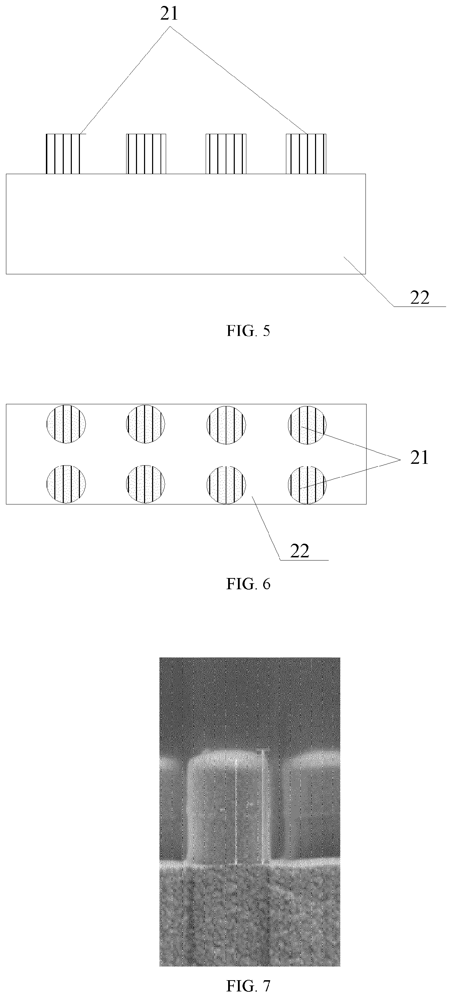

In the examples of the present application, the shape of the geometric structure on the surface of the chip substrate can be a protrusion formed on the surface of the chip substrate, i.e., a three-dimensional structure protruding from the surface of the chip substrate, or may be a depression on the surface of the chip substrate, i.e., a depressed cavity structure formed on the substrate. Illustratively, when the geometric structure is a cylindrical depression, the schematic cross-sectional view and the schematic top view of the chip substrate are respectively shown in FIGs. 1 and 2, where 11 is the cylindrical depression, and 12 is the chip substrate. The cross-sectional view and the plan view are respectively shown in FIG. 3 and FIG. 4; when the geometric structure is a cylindrical protrusion, the schematic cross-sectional view and the schematic top view of the chip substrate are respectively shown in FIGs. 5 and 6, where 21 is the cylindrical protrusion, and 22 is the chip substrate; the cross-sectional view is shown in FIG. 7.

In the examples of the present application, the geometric structure is provided on at least one surface of the chip substrate.

In one embodiment, the geometric structure of the chip substrate is provided on one surface of the chip substrate. At this point, the material of the chip substrate may be selected from the above-listed materials, for example, glass; quartz; a silicon-based material such as silicon dioxide, polysilicon, single-crystal silicon, etc.; a polymer such as hexamethyldisilazane (HMDS), polyethylene (PE), polypropylene (PP), etc.; a metal, e.g., aluminum, etc. The chip substrate may be made of the above, or may be made of a composite consisting of two or more of the above materials. In some examples, the chip substrate may be provided as a single layer, and the single-layer chip substrate may be made of one material or a mixture of materials; the chip substrate may also be provided as a laminated structure, where each layer forming the laminated structure can be made of one material or made of a mixture of materials, and the geometric structure is provided on the surface layer of the laminated structure and can be depressed beneath the surface to at least one other laminated layer.

In another embodiment, the geometric structure of the chip substrate is provided on two surfaces of the chip substrate. The resultant chip substrate provides two surfaces for biochemical reactions and/or detections, such as nucleic acid sequencing, thus advantageously improving the throughput of the biochemical reactions and/or detections. In one example, the chip substrate includes a single layer, and the light transmittance of the single-layer chip substrate is less than 70% or the thickness of the single-layer chip substrate is 500 µm or greater, such that when an imaging device collects the optical signals from the two surfaces (especially the geometric structures) of the chip substrate, the interference between the optical signals of the two surfaces is reduced. The single-layer chip substrate can be made of an opaque material, which may be a silicon-based material such as silicon dioxide, polysilicon, single-crystal silicon, etc.; or a metal, such as aluminum, etc. The single-layer chip substrate may also be made of various mixed materials, and the light transmittance of the film layer formed by the mixed materials is less than 70% or the thickness of the film layer formed by the mixed materials is 500 µm or greater. For example, a silicon-based material is mixed with other materials in a proper ratio to give the chip substrate. In another example, the chip substrate is a laminated structure, including at least one layer having a light transmittance of less than 70% or a layer having a thickness of 500 µm or greater, so as to reduce the mutual interference of optical signals generated from two surfaces (especially geometric structures) of the chip substrate. Illustratively, the chip substrate is a laminated structure including two layers. In one case, one layer is a layer having a light transmittance of greater than 95%, and the other layer is a layer having a light transmittance of less than 70%; the surfaces of the two layers away from each other are respectively provided with the geometric structure. In another case, both layers are those having a light transmittance of less than 70%; the surfaces of the two layers away from each other are respectively provided with the geometric structure. In still another case, both layers are those having a light transmittance of greater than 95%, and the overall thickness of the two layers is 500 µm or greater; the surfaces of the two layers away from each other are respectively provided with the geometric structure.

In the examples of the present application, the dimension of the geometric structure meets the detection requirement of the biomolecule. Specifically, the geometric structure has a height of 10 nm to 100 µm in a direction perpendicular to the surface of the chip and a maximum dimension of 10 nm to 100 µm in a direction parallel to the surface of the chip. The height of the geometric structure in the direction perpendicular to the surface of the chip may be interpreted as the depth (when the geometric structure is a depression) or the height (when the geometric structure is a protrusion) of the geometric structure. The maximum dimension in the direction parallel to the surface of the chip may be interpreted as the greatest distance between any two points on the circumference of a cross-section (a cross-section in a direction parallel to the surface of the chip substrate) of the geometric structure. In some examples, the geometric structure has a height of 50 nm to 10 µm, preferably 100 nm to 1 µm, and more preferably 200 nm to 800 nm, in a direction perpendicular to the surface of the chip and a maximum dimension in a direction parallel to the surface of the chip of 50 nm to 10 µm, preferably 100 nm to 1 µm, and more preferably 200 nm to 800 nm. In the examples of the present application, the distance between adjacent geometric structures, i.e., the center-to-center distance of adjacent geometric structures, is 10 nm to 100 µm. In some examples, the distance between adjacent geometric structures is 50 nm to 10 µm, preferably 100 nm to 1 µm, and more preferably 200 nm to 800 nm.

In one example, the geometric structure has a height of 300 nm to 500 nm in a direction perpendicular to the surface of the chip and a maximum dimension of 300 nm to 500 nm in a direction parallel to the surface of the chip; the distance between adjacent geometric structures is 300 nm to 700 nm. When used for sequencing, the chip provided by the method can improve the sequencing quality and feature sequencing results with high repeatability and good stability and resistance to multiple cycles of reagent scouring.

In some embodiments, the geometric structures are in a regular arrangement on the surface of the chip substrate, or in other words, the geometric structures are regularly arranged to form a pattern of a certain shape. In some examples, the surface is provided with at least one array unit having the regularly arranged geometric structures. Specifically, the array unit is a square array unit. That is, the pattern formed by a set of a plurality of geometric structures is a square. Illustratively, when the geometric structures are arranged in rows and columns, the distance between adjacent rows is equal to the distance between adjacent columns, and the set of the plurality of geometric structures includes the same number of rows and columns. The array unit is a rectangular array unit. That is, the pattern formed by a set of a plurality of geometric structures is a rectangle. Illustratively, when the geometric structures are arranged in rows and columns, the distance between adjacent rows is equal to the distance between adjacent columns, and the set of the plurality of geometric structures includes the different numbers of rows and columns. The array unit is a honeycomb array unit. That is, the pattern formed by a set of a plurality of geometric structures is a honeycomb shape. In other words, the plurality of geometric structures are regularly arranged such that the set of geometric structures form a honeycomb shape. Certainly, one surface may include two or more of the square array unit, the rectangular array unit, and the honeycomb array unit. In one example, one surface of the chip substrate may include one or more sets of geometric structures, and in each set, the geometric structures are arranged according to the same rule. In another example, one surface of the chip substrate includes one or more sets of geometric structures, and the arrangement of geometric structures in at least one set is different from that of geometric structures in another set. That is, one chip substrate includes a combination of multiple arrangements, e.g., by splicing or nesting.

In one embodiment, the geometric structure is a circular well structure with a depth of 300 nm to 500 nm, a diameter of 300 nm to 500 nm, and a distance between adjacent circular wells of 300 nm to 900 nm.

In some embodiments, the chip may further include an encapsulation layer arranged on the chip substrate and configured for encapsulating the surface of the chip substrate provided with the geometric structure, so as to protect the chip substrate and form a reaction region through which a solution may flow.

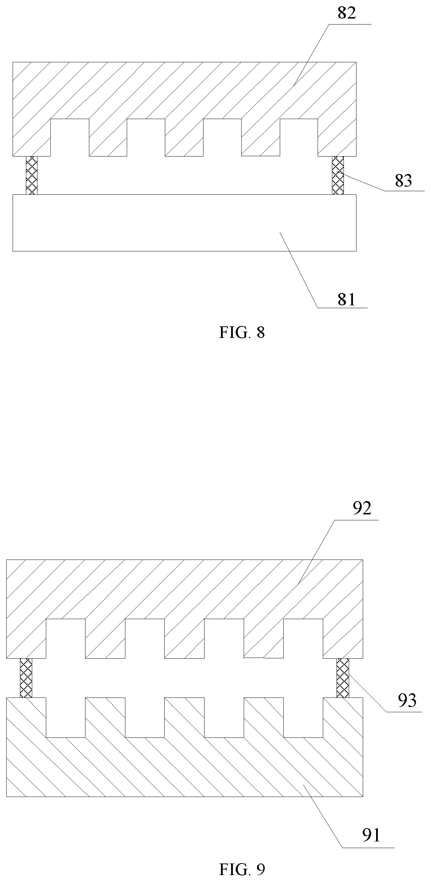

In one embodiment, the chip includes a first cover plate bonded to the surface of the chip substrate provided with the geometric structure. The first cover plate is made of a material with a light transmittance of 95% or greater, such that light can reach the surface of the chip substrate provided with the geometric structure through the first cover plate. The material of the first cover plate may be identical to or different from that of the chip substrate, which is not limited in the present application. Illustratively, the material of the cover plate may be glass, quartz, a polymer film, etc. In some examples, as shown in FIGs. 8 and 9, the geometric structure of the chip substrate 82 is formed on one surface of the chip substrate 82, and the first cover plate 81 is bonded to the surface of the chip substrate 82 provided with the geometric structure, so as to implement the encapsulation of the chip. The first cover plate 81 may be a patterned material or an unpatterned material.

In one example, as shown in FIG. 8, the first cover plate 81 is a normal cover plate without a geometric structure, that is, the two surfaces of the cover plate are untreated flat surfaces. At this point, the surface of the first cover plate 81 facing the chip substrate 82 is a flat surface. In one example, a glue layer 83 is arranged between the first cover plate 81 and the chip substrate 82 for adhering the first cover plate 81 and the chip substrate 82. The glue layer 83 has a channel arranged in a length direction of the chip; the geometric structure is arranged on the surface of the chip substrate 82 in a region where the channel is located, and the shapes and/or arrangements of the geometric structures in different channels on the same surface are identical or different.

In some other examples, as shown in FIG. 9, the first cover plate 91 is provided with regularly arranged geometric structures on one surface. The geometric structure of the first cover plate 91 is arranged opposite to the geometric structure of the chip substrate 92. That is, the surface of the first cover plate 91 facing the chip substrate 92 is provided with regularly arranged geometric structures. At this point, one chip is provided with two surfaces for sequencing, thus improving the sequencing throughput. In this example, the geometric structure of the first cover plate 91 may be identical to or different from that of the chip substrate 92. The details are not recited here for brevity. **In** one example, a glue layer 93 is arranged between the first cover plate 91 and the chip substrate 92 for adhering the first cover plate 91 and the chip substrate 92. The glue layer 93 has a channel arranged in a length direction of the chip; the geometric structure is arranged on the surfaces of the chip substrate 92 and the first cover plate 91 in a region where the channel is located, and the shapes and/or arrangements of the geometric structures in different channels on the same surface are identical or different.

In another embodiment, the chip substrate is provided with regularly arranged geometric structures on the two surfaces, and the geometric structures on the two surfaces may be in identical or different arrangements. The arrangement of the geometric structures on the two surfaces of the chip substrate is not recited here for brevity. At this point, the light transmittance of the chip substrate is less than 70% or the thickness of the chip substrate is 500 µm or greater, such that when an imaging device collects the optical signals from the two surfaces (especially the geometric structures) of the chip substrate, the interference between the optical signals of the two surfaces is reduced. In this embodiment, the cover plate is bonded to at least one surface of the chip substrate provided with the geometric structure, so as to implement the encapsulation of the chip. In some examples, the chip includes a first cover plate and a second cover plate respectively bonded to the two surfaces of the chip substrate, where the first cover plate and the second cover plate are made of a material having a light transmittance of 95% or greater, such that light can reach the two surfaces of the chip substrate provided with the geometric structure through the first cover plate and the second cover plate, respectively.

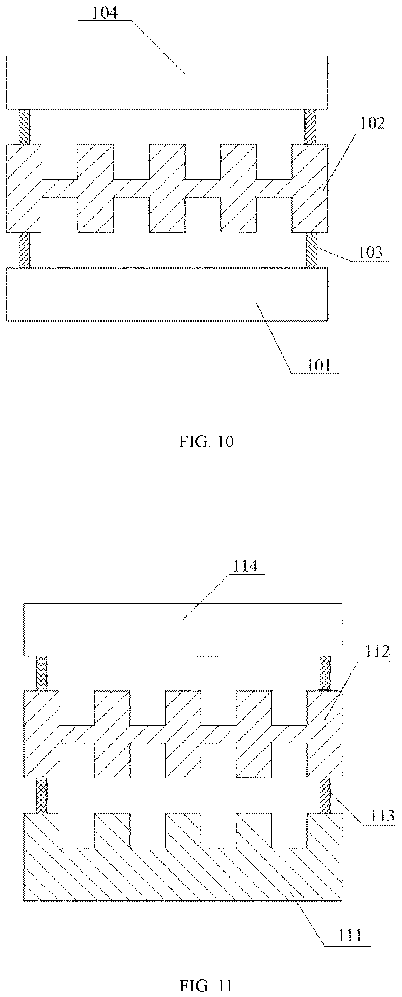

In some examples, the cover plates are normal cover plates without a geometric structure, that is, the two surfaces of the cover plates are untreated flat surfaces. In one example, as shown in FIG. 10, the first cover plate 101 and the second cover plate 104 are respectively bonded to two surfaces of the chip substrate 102 provided with the geometric structure, and the first cover plate 101 and the second cover plate 104 are normal cover plates without a geometric structure, that is, the two surfaces of the first cover plate 101 and the second cover plate 104 are flat surfaces. In one example, the glue layers 103 are arranged between the first cover plate 101 and the chip substrate 102 and between the second cover plate 104 and the chip substrate 102 for adhering the first cover plate 101 and the chip substrate 102, and the second cover plate 104 and the chip substrate 102. The glue layers 103 have channels arranged in a length direction of the chip; the geometric structures are arranged on the surfaces of the chip substrate 102 in regions where the channels are located, and the shapes and/or arrangements of the geometric structures in different channels on the same surface are identical or different.

In some examples, the first cover plate and/or the second cover plate are cover plates provided with the geometric structure on one surface.

In one example, as shown in FIG. 11, the first cover plate 111 and the second cover plate 114 are respectively bonded to two surfaces of the chip substrate 112 provided with the geometric structure, where the first cover plate 111 is a cover plate provided with the geometric structure, and the geometric structure of the first cover plate 111 is arranged opposite to the geometric structure of the chip substrate 112; the second cover plate is a normal cover plate without a geometric structure, and the two surfaces of the second cover plate 114 are flat surfaces. It will be appreciated that the geometric structure of the first cover plate 111 may be identical to or different from that of the chip substrate 112. In one example, the glue layers 113 are arranged between the first cover plate 111 and the chip substrate 112 and between the second cover plate 114 and the chip substrate 112 for adhering the first cover plate 111 and the chip substrate 112, and the second cover plate 114 and the chip substrate 112. The glue layers 113 have channels arranged in a length direction of the chip; the geometric structures are arranged on the surfaces of the chip substrate 112 and the first cover plate 111 in regions where the channels are located, and the shapes and/or arrangements of the geometric structures in different channels on the same surface are identical or different.

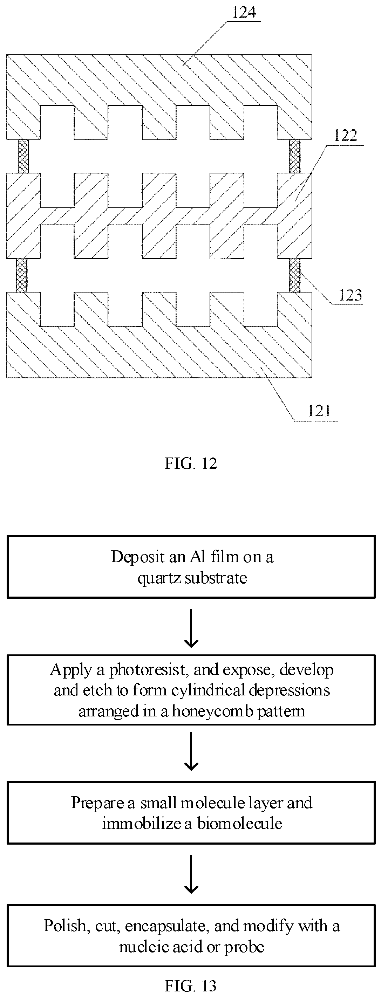

In one example, as shown in FIG. 12, the first cover plate 121 and the second cover plate 124 are respectively bonded to two surfaces of the chip substrate 122 provided with the geometric structure, where the two cover plates are those provided with the geometric structure, and the geometric structures of the cover plates are arranged opposite to the geometric structure of the chip substrate 122 to form a double-chip-like, laminated structure. It will be appreciated that the geometric structure of each cover plate may be identical to or different from that of the chip substrate; the geometric structures of the two cover plates may be identical or different. In one example, the glue layers 123 are arranged between the first cover plate 121 and the chip substrate 122 and between the second cover plate 124 and the chip substrate 122 for adhering the first cover plate 121 and the chip substrate 122, and the second cover plate 124 and the chip substrate 122. The glue layers 123 have channels arranged in a length direction of the chip; the geometric structures are arranged on the surfaces of the chip substrate 122, the first cover plate 121, and the second cover plate 122 in regions where the channels are located, and the shapes and/or arrangements of the geometric structures in different channels on the same surface are identical or different.

When the chip is used for sequencing, by arranging the regularly arranged geometric structures on the surface of the cover plate facing the chip substrate, the above embodiment can increase the reaction and/or detection sites for nucleic acid molecules on the surface of the chip, which is beneficial to increasing the sequencing throughput. It will be appreciated that when the cover plate is provided with the regularly arranged geometric structures, the reaction and/or detection sites for the nucleic acid molecules on the surface of the chip can be increased, thus facilitating the improvement of the sequencing throughput. Such a cover plate also possesses the functionality of the chip substrate, and thus may also be referred to as a chip substrate.

In some embodiments, the chip includes a polymer layer arranged on the surface provided with the geometric structure. Since the chip substrate is provided with the geometric structure on at least one surface, the polymer layer is arranged at least on the surface of the chip substrate provided with the geometric structure. When the two surfaces of the chip substrate are provided with the geometric structure, both surfaces are provided with the polymer layer. Similarly, when the cover plate, including the first cover plate and the second cover plate, is provided with the geometric structure on one surface, the polymer layer is correspondingly arranged on the surface of the cover plate provided with the geometric structure. In this embodiment, the surface of the geometric structure refers to a region where the geometric structure is parallel to the surface of the chip substrate. When the geometric structure is a recess structure, the surface of the geometric structure is the bottom surface of the recess structure. At this point, the polymer layer is arranged on the bottom surface of the recess structure. It is possible that the polymer layer may extend to the inner wall surface of the recess structure. When the geometric structure is a protrusion structure, the surface of the geometric structure is a top surface of the protrusion structure. At this point, the polymer layer is arranged on the top surface of the protrusion structure.

The polymer layer provides a capture site or a detection site for the analysis and/or detection of nucleic acid molecules by binding to a ligand for nucleic acid molecules, such as a nucleic acid probe, an antigen/antibody, a nucleic acid-protein complex, etc. As such, in the examples of the present application, the size and the arrangement of the geometric structure are such set that the nucleic acid molecules are immobilized on the surface of the chip in a certain arrangement density and arrangement manner. That is, the nucleic acid molecules form the detection or analysis sites in a certain density and arrangement manner, which is beneficial to acquiring and positioning the information generated during the reaction and/or detection of the nucleic acid molecules in the later stage, and is helpful to improve the stability of the detection results for the nucleic acid molecules. In some examples, the polymer layer is formed from a polymer containing a first functional group selected from one or more of sulfydryl, formyl, hydroxyl, carboxyl, amino, alkenyl, alkynyl, and azido. These functional groups are chemically reactive or adsorptive, and can react with other functional groups with reactivity to bind to a nucleic acid molecule or a ligand for nucleic acid molecules, such as a nucleic acid probe, an antigen/antibody, or a nucleic acid-protein complex. Illustratively, the polymer constituting the polymer layer may be, but is not limited to, polyethylene glycol, polypropylene, polyacrylic acid, poly(methyl methacrylate), etc. In the examples of the present application, the polymer formed on the surface of the chip provided with the geometric structure can optimize the environment for the chip sequencing reaction. Particularly, the formed polymer layer has a high transparency and a low optical background in a visible light wave band of 400 nm-700 nm.

The thickness of the polymer layer is not particularly limited in the present application, as long as the nucleic acid molecule or the ligand for nucleic acid molecules can be immobilized.

In this embodiment, since the material of the chip substrate and/or the cover plate are less reactive, in some examples, the polymer layer is bonded to the surface of the chip substrate and/or the cover plate via a coupling molecule. In one example, the coupling molecule has the following formula: XₐSi(Y_{b})M_{c}Z, where X and Z are reactive groups for coupling the chip substrate and the polymer, respectively.

Among these, X is H, F, Cl, Br, I, C₁₋₆ alkyl, C₁₋₆ haloalkyl, or C₁₋₆ alkoxy, for example, Cl, CH₃O-, CH₃CH₂O-, etc.; Y is selected from C₁₋₁₀ alkyl, e.g., CH₃-, CH₃CH₂-, etc.; M_{c} is selected from C₁₋₅₀ alkylene, -(OCH₂CH₂)_{c}-, and -(CH(OH)CH₂-)_{c}; Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl; the C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl are substituted with at least one R^{a}, and each R^{a} is independently selected from halogen, nitro, ester group, amino, carboxyl, hydroxyl, azido, and epoxy group; a, b, and c are integers meeting the following conditions: 0 ≤ a ≤ 3, 0 ≤ b ≤ 3, and a + b = 3; 1 ≤ c ≤ 50.

Illustratively, the coupling molecule may be at least one of the following structures: 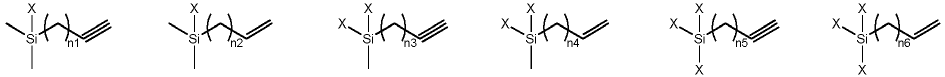 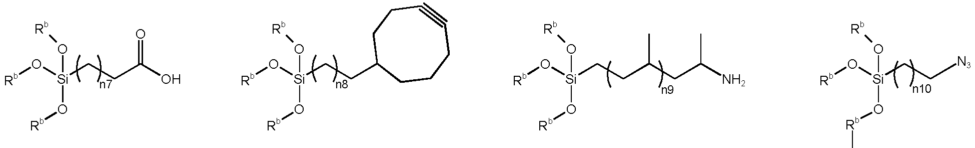
where X is a halogen; for example, X is F, Cl, Br, or I;
R^{b} is -CH₃, -C₂H₅, -C₃H₇, or -C₄H₉;
n1 is selected from integers of 2 to 10; for example, n1 is 2, 3, 4, 5, 6, 7, 8, 9, or 10;
n2 is selected from integers of 2 to 10; for example, n2 is 2, 3, 4, 5, 6, 7, 8, 9, or 10;
n3 is selected from integers of 2 to 10; for example, n3 is 2, 3, 4, 5, 6, 7, 8, 9, or 10;
n4 is selected from integers of 2 to 10; for example, n2 is 2, 3, 4, 5, 6, 7, 8, 9, or 10;
n5 is selected from integers of 2 to 10; for example, n2 is 2, 3, 4, 5, 6, 7, 8, 9, or 10;
n6 is selected from integers of 2 to 10; for example, n2 is 2, 3, 4, 5, 6, 7, 8, 9, or 10;
n7 is selected from integers of 1 to 10; for example, n2 is 1, 2, 3, 4, 5, 6, 7, 8, 9, or 10;
n8 is selected from integers of 5 to 10; for example, n2 is 5, 6, 7, 8, 9, or 10;
n9 is selected from integers of 1 to 10; for example, n2 is 1, 2, 3, 4, 5, 6, 7, 8, 9, or 10;
n10 is selected from integers of 1 to 10; for example, n2 is 1, 2, 3, 4, 5, 6, 7, 8, 9, or 10.

In some embodiments, the chip further includes a first film layer arranged between the chip substrate and/or the cover plate and the polymer layer. Specifically, the first film layer is optionally arranged on the surfaces of the chip substrate and the cover plate provided with the geometric structures, and the material of the first film layer is different from that of the surface bonded thereto, so as to reduce surface defects of the chip substrate and/or the cover plate and improve the uniformity of the chip surface. Furthermore, in the preparation process of the chip, considering the feasibility of the process, it is a tendency to form the polymer layer by reaction on the entire surface of the chip substrate and/or the cover plate and then treat the polymer layer on the surfaces of the chip substrate and/or the cover plate to retain only the polymer formed in the geometric structures, so as to meet the requirement of detecting the optical signal generated by the reaction of the nucleic acid molecules. In some examples, the polymer is coupled within the geometric structure by a selective modification process. In some examples, the polymer outside the geometric structure is removed by a polishing process. At this point, the polishing endpoint may be determined by arranging the first film layer on the surface of the chip substrate and/or the cover plate, and indicating the degree of polishing reaction by detecting the physicochemical properties of the first film layer.

In this embodiment, the first film layer includes, but is not limited to, a metal film, such as Al film, Au film, etc.; a metal oxide film, such as Al₂O₃ film, etc.; an inorganic oxide film, such as SiO₂ film, etc. The first film layer may be one or more of the above, preferably one or more of Al film, Al₂O₃ film, and SiO₂ film. Illustratively, when the SiO₂ film is used as the first film layer, the SiO₂ film can stabilize the properties of the surface of the chip, and specifically, avoid the reaction between materials, reagents or the like introduced by the reaction and/or detection with the chip substrate and thus the generation of bubbles. In addition, the SiO₂ can also improve the bonding firmness of the polymer and reduce the risk of the polymer layer falling off from the chip surface. In some examples, the first film layer has a thickness of 10 to 1000 nm, preferably 15 to 800 nm, more preferably 20 to 500 nm, and most preferably 20 to 200 nm. Within this thickness range, the first film layer can exert the above effects. For the first film layer having an excessive thickness, e.g., greater than 1500 nm, the light intensity of the base collected from the geometric structure of the chip provided with the first film layer is reduced during the sequencing as compared with the chip without the first film layer, thereby affecting the determination of the sequencing signals. Illustratively, the thickness of the first film layer may be 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 110 nm, 120 nm, 130 nm, 140 nm, 150 nm, 160 nm, 170 nm, 180 nm, 190 nm, 200 nm, 250 nm, 300 nm, 400 nm, 500 nm, 600 nm, 700 nm, 800 nm, 900 nm, 1000 nm, etc.

In some examples, the chip substrate and the cover plate are bonded by an adhesive material, and the adhesive material forms a channel structure through hollowing to provide a reaction region for the chip substrate. The adhesive material may be a binder, a double-sided tape, or a light-curable material such as a UV glue, etc. Considering the influence on the reagents in the sequencing process, a binder with no influence on the nucleic acid molecules and the reagents used in the sequencing process and stable in the reagents used in the sequencing process, should be selected. Illustratively, the adhesive material is a UV-curable adhesive material, a thermosetting adhesive material, a pressure-sensitive adhesive material, etc, which, however, is not limited thereto.

In some examples, the chip has at least one channel arranged in a length direction, for example, by forming one or more channels between the chip substrate and the encapsulation layer, to achieve fluid communication functionality. Specifically, the geometric structure is arranged on the surface of the chip substrate in a region where the channel is located, and the solution reacts with the nucleic acid molecules immobilized on the geometric structure when flowing through the channel. It will be appreciated that the shapes and/or arrangements of the geometric structures in different channels are identical or different.

By forming the regularly arranged geometric structures on the substrate, the chip provided by the examples of the present application can improve the stability of sequencing and the accuracy of sequencing results. Moreover, the regularly arranged geometric structures can improve the arrangement density of the geometric structures on the chip and thus the detection throughput; the detection sites corresponding to the geometric structures can be positioned through the regularly arranged geometric structures, thereby facilitating the synchronous detection of multiple nucleic acid molecules on the surface of the same chip.

The chip provided by the examples of the present application may be prepared by the following method, which, however, is not limited thereto.

Correspondingly, the examples of the present application also provide a method for preparing a chip, including:
a) forming regularly arranged geometric structures on at least one surface of a substrate,

where the geometric structure has a height of 10 nm to 100 µm in a vertical direction and a maximum dimension of 10 nm to 100 µm in a horizontal direction;
the distance between adjacent geometric structures is 10 nm to 100 µm.

In step a), the substrate is a target substrate requiring the arrangement of the geometric structure and at least includes a chip substrate. In some examples, the target substrate requiring the arrangement of the geometric structure is a chip substrate, and at this point, the substrate is the chip substrate. In some examples, the target substrate requiring the arrangement of the geometric structure is a cover plate, and at this point, the substrate is the cover plate. In some examples, the target substrate requiring the arrangement of the geometric structure includes a chip substrate and a cover plate, and at this point, the substrate includes the chip substrate and the cover plate. The materials of the substrate can be selected according to the above materials of the chip substrate and the cover plate, which are not recited here.

In the examples of the present application, the regularly arranged geometric structures are prepared on the surface of the substrate, and the shape, dimension parameter, and arrangement rule of the geometric structure are as described above, which are not recited here.

According to the examples of the present application, the regularly arranged geometric structures can be prepared on the surface of the substrate by means of photoetching, nano-imprinting, screen printing, etc.

Illustratively, the preparation of the geometric structure by photoetching includes: cleaning and drying the substrate, applying a photoresist, and exposing, developing and etching to give the regularly arranged geometric structures, i.e., to directly acquire a pattern. The preparation may also include: applying a photoresist on other substrates, exposing, developing and etching to give the regularly arranged geometric structures, and transferring the geometric structure to a target substrate to form a pattern. The reagents, methods, and the like for photoetching are not particularly limited in the examples of the present application, where the method for etching may be dry etching, such as plasma etching, electron beam etching, laser etching and the like, and may also be wet etching, such as acid or base etching, oxidation-reduction etching and the like.

Illustratively, the preparation of the geometric structure by nano-imprinting technology may include: preparing a template of a required pattern, applying a photoresist on the substrate, pressing the template on the surface of the photoresist, and transferring the pattern onto the photoresist by pressurizing to give the regularly arranged geometric structures, i.e., to directly acquire the pattern. The preparation may also include: preparing a template of a required pattern on a substrate, applying a photoresist on a second substrate, pressing the template on the surface of the photoresist, transferring the pattern onto the photoresist by pressurizing, curing the photoresist to give the regularly arranged geometric structures, and transferring the geometric structure onto the chip substrate by etching to form the pattern. The reagents, methods, and the like for nano-imprinting are not particularly limited in the examples of the present application, where the method for etching may be dry etching, such as plasma etching, electron beam etching, laser etching and the like, and may also be wet etching, such as acid or base etching, oxidation-reduction etching and the like.

Illustratively, the preparation of the geometric structure by screen printing specifically includes: preparing the regularly arranged geometric structures on the chip substrate by screen printing, i.e., to directly acquire the pattern. The preparation may also include: preparing the regularly arranged geometric structures on a substrate by screen printing, and transferring the geometric structures onto the chip substrate by etching to form the pattern. The reagents, methods, and the like for screen printing are not particularly limited in the examples of the present application, where the method for etching may be dry etching, such as plasma etching, electron beam etching, laser etching and the like, and may also be wet etching, such as acid or base etching, oxidation-reduction etching and the like.

In some embodiments, the pattern may be produced by one of the methods described above, or by a combination of two or more methods. After the geometric structure is acquired, it may be post-treated to give the final patterned surface. In some specific embodiments, the post-treatment includes, but is not limited to, an enhancement treatment, such as physical vapor deposition (PVD), chemical vapor deposition (CVD), electroplating, etc.; or a removal treatment, such as plasma ashing, solution etching, dry oxidation, etc. The specific process parameters of the above treatment are not particularly limited in the examples of the present application, and can be selected by those skilled in the art as needed.

In some embodiments, the method of preparing the chip further includes: preparing a first film layer on the surface of the chip substrate provided with the geometric structure, where the first film layer and the substrate are made of different materials. The parameters and materials of the first film layer and the substrate are described above and are not recited here. In some examples, the first film layer is selected from one or more of a metal film, a metal oxide film, and an inorganic oxide film. In some examples, the first film layer is made of at least one of Au, Al, and SiO₂. In some examples, the first film layer has a thickness of 10 to 1000 nm.

According to the examples of the present application, the first film layer may be formed on the substrate by deposition before the geometric structure is prepared; the first film layer may also be deposited after the geometric structure is prepared and formed on the chip substrate. That is, the first film layer may be prepared before step a) or after step a). The deposition method is not particularly limited in the examples of the present application, and may be physical vapor deposition (PVD), chemical vapor deposition (CVD), electroplating, oxidation, etc.

In some specific embodiments, the method further includes, after forming the regularly arranged geometric structures:
b) forming a polymer layer in the geometric structure.

After the regularly arranged geometric structures are formed on the substrate, the surface is modified to provide capture or detection sites.

In some examples, the chip substrate is preferably pre-treated before forming the polymer layer. In some specific embodiments, the pre-treatment includes cleaning and plasma activation. The cleaning may be a solution cleaning, followed by plasma activation. In some specific embodiments, the gas used for plasma activation includes, but is not limited to, air, nitrogen, argon, hydrogen, carbon tetrafluoride, and the like, and may be a mixed gas of one or more of them. In some specific embodiments, the power of the plasma activation is 10 W to 2 kW.

In one embodiment, the geometric structure is a depression formed on the surface of the chip substrate and/or the cover plate, and at this point, b) includes:
bonding a polymer containing a first functional group to the surface provided with the geometric structure; and
polishing the polymer to remove the polymer outside the region where the geometric structure is located, so as to form the polymer layer on the geometric structure.

It will be appreciated that this embodiment includes the case where the polymer is bonded to the surface provided with the geometric structure via a coupling molecule. In this way, the polymer at the periphery of the geometric structure can be removed to reduce the nonspecific adsorption on the surface of the chip.

In the examples of the present application, the polymer is polished to remove the polymer outside the region where the geometric structure is located, where the removal method is not particularly limited, and includes but is not limited to solution cleaning, chemical polishing, mechanical polishing, chemomechanical polishing, etc. Specifically, the solution cleaning is to remove the polymer molecule on the periphery of the geometric structure by using a reagent capable of dissolving and removing the polymer molecule, such as an acid, a base, a solvent, a surfactant, etc, and is preferably performed under ultrasonic conditions or performed by using a flushing manner. In some embodiments, the polymer is removed by polishing, which may be a chemical polishing based on chemical reaction removal, a mechanical polishing based on mechanical action removal, or a combination thereof. In the examples of the present application, the polishing may be performed by using chemical polishing reagents, such as one or more of an acid, a base, an organic solvent, and a surfactant, or may be a mechanical polishing using, e.g., one or more reagents of diamond, silica sol, calcium carbonate, cerium oxide, and zirconium oxide, etc., as the polishing reagent.

In the technical scheme provided by the present application, when the substrate is treated by polishing, the chip substrate is preferably a structure including the substrate and the first film layer. At this point, the first film layer can be used as a polishing endpoint indicator for indicating the endpoint of polishing, thus facilitating the control of the polishing on the surface to give a chip with a stable and uniform surface, and thus further facilitating the detection stability.

In another embodiment, the geometric structure is a protrusion formed on the surface of the chip substrate and/or the cover plate, and at this point, a) includes: bonding the polymer containing the first functional group to the surface of the substrate. The substrate with the polymer formed on the surface is then patterned to give the regularly arranged geometric structures on the surface.

According to the two embodiments, in some examples, the first functional group is selected from one or more of sulfydryl, formyl, hydroxyl, carboxyl, amino, alkenyl, alkynyl, and azido.

According to the two embodiments, in some examples, the substrate is treated with the coupling molecule, such that the coupling molecule is formed on the surface of the substrate to be treated or the surface provided with the geometric structure, and the coupling molecule is modified with the polymer to form the polymer layer. The chip substrate is preferably pre-treated before the coupling molecule is bonded to the surface of the chip substrate, and in some specific embodiments, the pre-treatment includes cleaning and plasma activation. The cleaning may be a solution cleaning, followed by plasma activation. In some specific embodiments, the gas used for plasma activation includes, but is not limited to, air, nitrogen, argon, hydrogen, carbon tetrafluoride, and the like, and may be a mixed gas of one or more of them. In some specific embodiments, the power of the plasma activation is 10 W to 2 kW. After the pre-treatment, the coupling molecule can be bonded by a method such as chemical vapor deposition (CVD), physical vapor deposition (PVD), atomic layer deposition, magnetron sputtering, a solution-based method, spin-coating, spraying, etc., so as to provide active sites for immobilizing the polymer.

The coupling molecule has the following formula: XₐSi(Y_{b})M_{c}Z, where X and Z are reactive groups for coupling the chip substrate and the polymer, respectively.

Among these, X is H, F, Cl, Br, I, C₁₋₆ alkyl, C₁₋₆ haloalkyl, or C₁₋₆ alkoxy, for example, Cl, CH₃O-, CH₃CH₂O-, etc.; Y is selected from C₁₋₁₀ alkyl, e.g., CH₃-, CH₃CH₂-, etc.; M_{c} is selected from C₁₋₅₀ alkylene, -(OCH₂CH₂)_{c}-, and -(CH(OH)CH₂-)_{c}; Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl; the C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl are substituted with at least one R^{a}, and each R^{a} is independently selected from halogen, nitro, ester group, amino, carboxyl, hydroxyl, azido, and epoxy group; a, b, and c are integers meeting the following conditions: 0 ≤ a ≤ 3, 0 ≤ b ≤ 3, and a + b = 3; 1 ≤ c ≤ 50.

In some possible embodiments, the polymer may be bonded to the coupling molecule by a solution-based method, spin coating, screen printing, spraying, etc. In some specific embodiments, the temperature for modifying the coupling molecule with the polymer to form the polymer layer is 25 to 100 °C and the time is 1 h to 48 h.

In some examples, after forming the polymer layer, the polymer and coupling molecules at the periphery of the geometric structure are removed by post-treatment to reduce the non-specific adsorption on the surface of the biochip. In the examples of the present application, the removal method is not particularly limited, and includes but is not limited to solution cleaning, chemical polishing, mechanical polishing, chemomechanical polishing, etc. Specifically, the solution cleaning is to remove the coupling molecule on the periphery of the geometric structure by using a reagent capable of dissolving and removing the coupling molecule, such as an acid, a base, a solvent, a surfactant, etc., and is preferably performed under ultrasonic conditions or performed by using a flushing manner. In some embodiments, the coupling molecule is removed by polishing, which may be a chemical polishing based on chemical reaction removal, a mechanical polishing based on mechanical action removal, or a combination thereof. In the examples of the present application, the polishing may be performed by using chemical polishing reagents, such as one or more of an acid, a base, an organic solvent, and a surfactant, or may be a mechanical polishing using, e.g., one or more reagents of diamond, silica sol, calcium carbonate, cerium oxide, and zirconium oxide, etc., as the polishing reagent.

Similarly, when the chip substrate is treated by polishing, the chip substrate is preferably a structure including the chip substrate and the first film layer. At this point, the first film layer can be used as a polishing endpoint indicator for indicating the endpoint of polishing, thus facilitating the control of the polishing on the surface to evenly polish the surface and modify the coupling molecule, giving a chip with a stable and uniform surface, and further facilitating the detection stability.

In some specific embodiments, the method for preparing the chip further includes modifying the polymer layer with a capture molecule or a detection molecule or the like, such as a nucleic acid probe, an antigen/antibody, or a nucleic acid-protein complex. In the examples of the present application, the method for modification with the capture molecule or the detection molecule is not particularly limited, for example, by chemical coupling, including hydroxyl-carboxyl coupling, amino-carboxyl coupling, sulfydryl-sulfydryl coupling, click chemistry coupling, etc. In some specific embodiments, the capture molecule or the detection molecule may be modified after the post-treatment, or alternatively, the capture molecule or the detection molecule may be modified after forming the polymer layer, and then the extra polymer, capture molecule, or detection molecule may be removed by post-treatment.

In the examples of the present application, the chip includes a chip substrate, and at least the chip substrate is prepared by the method described above. In some specific embodiments, the method further includes: encapsulating the prepared chip substrate, and forming one or more flow channels on the geometric structure of the chip substrate to achieve fluid communication functionality, so as to achieve the flow reaction of different reagents on the chip in the detection processes and the resistance to multiple cycles of reagent scouring.

In some specific embodiments, the encapsulation material may be bonded to the chip substrate by a method including, but not limited to, thermal bonding, laser bonding, chemical-physical bonding, etc., where the chemical-physical bonding may be performed by bonding with a UV-curable adhesive material, a thermosetting adhesive material, a pressure-sensitive adhesive material, etc. In some specific embodiments, the encapsulation layer may be a cover plate provided with the geometric structure, which may be identical to or different from the geometric structure of the chip substrate, or may be a cover plate without a pattern.

In one example, as shown in FIG. 8, the first cover plate is a normal cover plate without a geometric structure, that is, the two surfaces of the first cover plate are untreated flat surfaces. The first cover plate is bonded to the surface of the chip substrate provided with the geometric structure, and in the bonding process, a fluid channel allowing a fluid to flow through the geometric structure is formed in the length direction of the surface of the chip provided with the geometric structure.

In one example, as shown in FIG. 9, the first cover plate is provided with regularly arranged geometric structures on one surface. The geometric structure of the first cover plate is arranged opposite to the geometric structure of the chip substrate. At this point, the surface of the first cover plate provided with the geometric structure is bonded opposite to the surface of the chip substrate provided with the geometric structure, and in the bonding process, a fluid channel allowing a fluid to flow through the geometric structure is formed in the length direction of the surface of the chip provided with the geometric structure. In this example, the geometric structure of the first cover plate may be identical to or different from that of the chip substrate. The details are not recited here for brevity.

In one example, as shown in FIG. 10, the first cover plate and the second cover plate are respectively bonded to two surfaces of the chip substrate provided with the geometric structure, and the first cover plate and the second cover plate are normal cover plates without a geometric structure, that is, the two surfaces of the first cover plate and the second cover plate are flat surfaces. The first cover plate and the second cover plate are respectively bonded to the two surfaces of the chip substrate provided with the geometric structure, and in the bonding process, a fluid channel allowing a fluid to flow through the geometric structure is formed in the length direction of the surface of the chip provided with the geometric structure.

In one example, as shown in FIG. 11, the first cover plate and the second cover plate are respectively bonded to two surfaces of the chip substrate provided with the geometric structure, where the first cover plate is a cover plate provided with the geometric structure, and the geometric structure of the first cover plate is arranged opposite to the geometric structure of the chip substrate; the second cover plate is a normal cover plate without a geometric structure, and the two surfaces of the second cover plate are flat surfaces. At this point, the surface of the first cover plate provided with the geometric structure is bonded opposite to the surface of the chip substrate provided with the geometric structure, and the second cover plate is bonded to the other surface of the chip substrate provided with the geometric structure; in the bonding process, a fluid channel allowing a fluid to flow through the geometric structure is formed in the length direction of the surface of the chip provided with the geometric structure. It will be appreciated that the geometric structure of the first cover plate may be identical to or different from that of the chip substrate.

In one example, as shown in FIG. 12, the first cover plate and the second cover plate are respectively bonded to two surfaces of the chip substrate provided with the geometric structure, where the two cover plates are those provided with the geometric structure, and the geometric structures of the cover plates are arranged opposite to the geometric structure of the chip substrate to form a double-chip-like, laminated structure. At this point, the surface of the first cover plate provided with the geometric structure is bonded opposite to the surface of the chip substrate provided with the geometric structure, and the surface of the second cover plate provided with the geometric structure is bonded opposite to the other surface of the chip substrate provided with the geometric structure; in the bonding process, a fluid channel allowing a fluid to flow through the geometric structure is formed in the length direction of the surface of the chip provided with the geometric structure. It will be appreciated that the geometric structure of each cover plate may be identical to or different from that of the chip substrate; the geometric structures of the two cover plates may be identical or different.

When the cover plate is a cover plate provided with the geometric structure, the cover plate may be prepared by the method described above. The material of the cover plate may be identical to or different from that of the chip substrate, and, as needed, includes, but is not limited to: glass; quartz; a silicon-based material such as silicon dioxide, polysilicon, single-crystal silicon, etc.; and a polymer such as HMDS, PE, PP, etc. Reference may be made to the description above regarding the chip. In some specific embodiments, the encapsulation may be performed between any of the steps of forming the coupling molecule, forming the polymer layer, post-treatment, and modification with the detection molecule or the capture molecule, without affecting subsequent procedures.

In some examples, the above steps are performed on a wafer-like chip substrate to improve production efficiency. Therefore, the chip can be diced to give chips of a desired size before encapsulation.

After the biochip is acquired, the biochip can be used for gene sequencing, providing improved detection throughput and stability and reliability for the sequencing structure.

Specifically, the nucleic acid molecule immobilized in the geometric structure of the chip is amplified to give an amplification cluster including 10-100000 copies of DNA molecules, and gene sequencing is performed on the amplification cluster. Since the geometric structure limits the amplification cluster in a certain space, the amplification cluster may present a certain shape, density and light intensity meeting the detection requirements, and thereby, improve the detection throughput and the stability and reliability of the sequencing structure. Moreover, by limiting the amplification cluster in the geometric structure of the chip provided by the embodiments of the present application, the stability of the amplification cluster in the sequencing process can be improved, allowing the amplification cluster to endure liquid environments including acidic reagents, basic reagents, organic solvents and the like, a broad reaction temperature range of 4-90 °C, 100 or more cycles of reagent scouring, etc., and to maintain good stability in such environments. Furthermore, the geometric structure, when used as the sequencing site, does not influence the biochemical functionality of biomolecules such as polymerase, amplification enzyme, and the like, and can reduce the adsorption of fluorescent dye and nucleic acid molecules.

In this example, the method for amplifying the immobilized nucleic acid molecules in the geometric structure of the chip is not strictly limited, and surface chain polymerase reaction (PCR) amplification such as bridge amplification, template-walking amplification, and the like, as well as rolling circle amplification, may be used, which, however, is not limited thereto.

The patterned biochip and the method for preparing same provided by the present application are further described below with reference to the following examples.

### Example 1

Probes were introduced into the biochips provided in Examples 1-4 and immobilized in the microwells via the polymer; a nucleic acid library (a nucleic acid sample of interest) was introduced and subjected to surface amplification to give an amplification cluster in the microwells;
Nucleic acid substrates ATCG containing a fluorophore and a polymerase were added for the base extension reaction. The influence signal of each base extension reaction was acquired, and the base type participating in each cycle of base extension reaction was determined through image analysis to determine the sequence of the nucleic acid library.

A silane molecular film layer with an amino functional group was formed on the surface of the quartz wafer, and a carboxyl-containing polymeric monomer was reacted on the silane molecular film layer to form the polymer film layer;
The coated chip substrate was cut and cleaned, and encapsulated with a cover plate with untreated surfaces to give the biochip.

### Example 2

The procedures are the same as those in Example 1 except that the depth of the circular well was 50 nm.

### Example 3

The procedures are the same as those in Example 1 except that the depth of the circular well was 500 nm.

### Example 4

The procedures are the same as those in Example 1 except that the depth of the circular well was 800 nm.

### Test Example 1

Amplification sequencing was performed on the biochips prepared in Examples 1 to 4 as follows: Probes were introduced into the biochips provided in Examples 1-4 and immobilized in the microwells via the polymer; a nucleic acid library (a nucleic acid sample of interest) was introduced and subjected to surface amplification to give an amplification cluster of the copies in the microwells;

Nucleic acid substrates ATCG containing a fluorophore and a polymerase were added for the base extension reaction. The influence signal of each base extension reaction was acquired, and the base type participating in each cycle of base extension reaction was determined through image analysis to determine the sequence of the nucleic acid library.

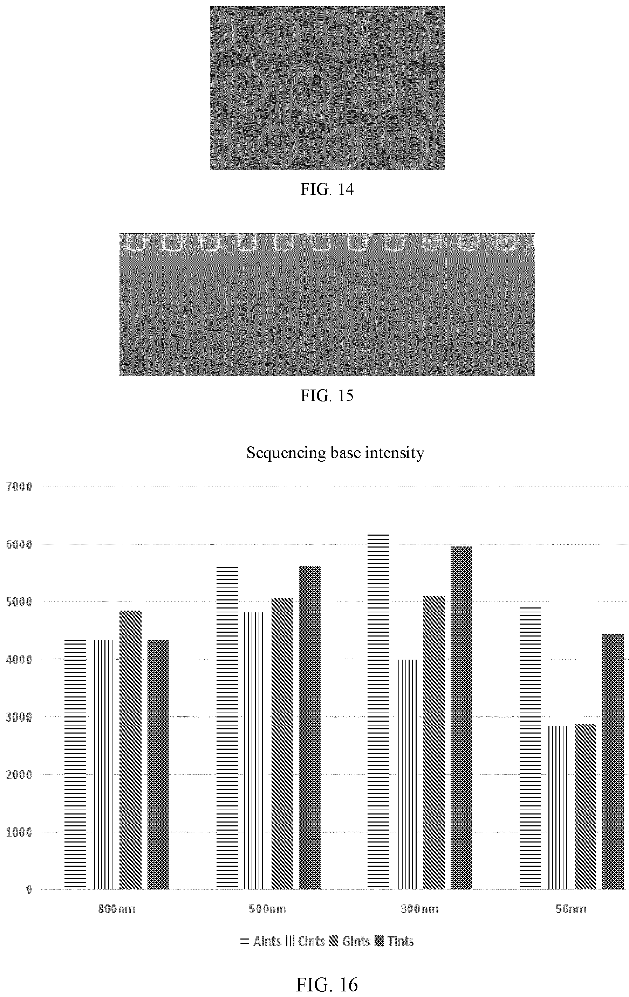

The light intensity of ATCG in the acquired fluorescent images of the chips provided in Examples 1-4 was summarized, and the results, as shown in FIG. 16, show that ATCG had a higher intensity in the fluorescence image in a well depth range of 300-800 nm, suggesting that the nucleic acid library had a higher copy number in microwells at a well depth of 300-800 nm.

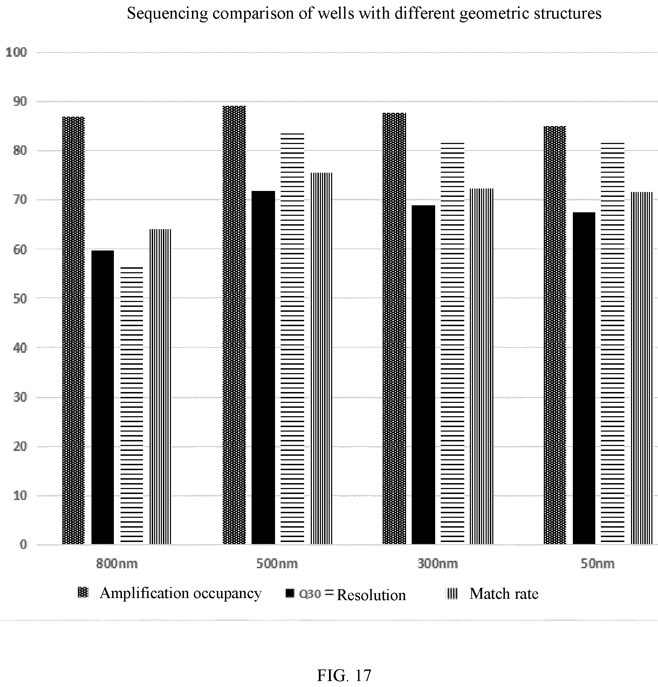

The summary of amplification occupancy, Q30, resolution, and match rate during the amplification on the chips provided in Examples 1-4 are shown in FIG. 17.

The amplification occupancy refers to the proportion of microwells participating in nucleic acid amplification in the patterned chip to all microwells in the patterned chip;
The Q30 refers to the percentage of bases having a Q value of 30 or greater, and the Q value is calculated by the formula: Q = -10log₁₀P, where P is the error rate; thus, the Q30 may also be interpreted as: the percentage of bases with a basecall error rate less than 0.1% in all sequenced bases; the Q30 reflects the sequencing quality, and a higher value indicates a better sequencing quality;
The resolution is the percentage of matches of nucleic acid molecules in each labeled sample to total nucleic acid molecules in the mixed sample during a mixed sequencing of the multiple samples; The match rate refers to the percentage of matches in sequences of interest the sample standard sequences.

FIG. 17 shows that ATCG had a higher intensity in the fluorescent image in a well depth range of 300-800 nm, and that the sequencing had a higher amplification occupancy and a higher accuracy in a well depth range of 50-500 nm.

### Example 5

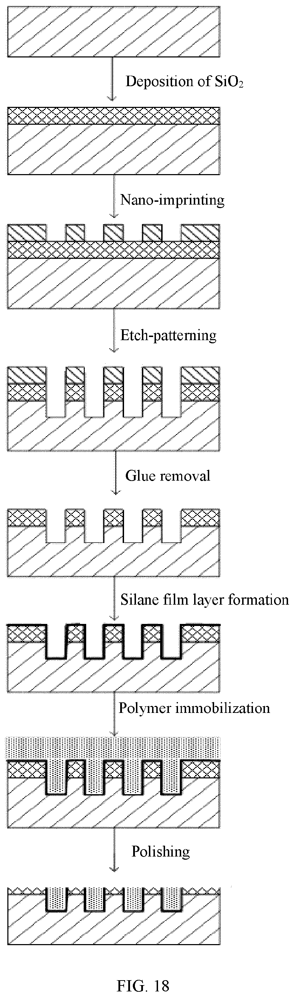

Now refer to FIG. 18 illustrating the method for preparing a biochip according to Example 5 of the present application:
Firstly, a silicon dioxide film with a thickness of 10 nm was formed on the surface of a silicon chip substrate by chemical vapor deposition; a patterned glue layer was then formed on the surface of the silicon dioxide film by nano-imprinting; the chip substrate was etched according to the pattern; after removing the glue layer, regularly arranged cylindrical depressions were formed on the silicon chip substrate, where the cylindrical depression had a depth of 300 nm, a cross-sectional diameter of 500 nm, and a distance (from center to center) between two adjacent cylindrical depressions of 3 µm; the cylindrical depressions were arranged in a honeycomb pattern; that is, the cylindrical depressions were arranged in rows and columns, the cylindrical depressions of two adjacent rows were not located in the same column, and the cylindrical depressions of alternate rows were located in the same column; the cylindrical depressions of two adjacent columns were not located in the same row, and the cylindrical depressions of alternate columns were located in the same row.

After forming the patterned chip substrate, a silane molecule film layer containing an amino functional group was formed in the cylindrical depression, and a polymer was then immobilized on the chip substrate by amino-carboxyl condensation; the bio macromolecule layer, the small molecule layer, and part of the silicon dioxide film outside the cylindrical depression were removed by polishing with the silicon dioxide film being the polishing endpoint; the polished chip substrate was cut, cleaned and encapsulated to give the biochip.

During the polishing, with the silicon dioxide film being the polishing endpoint, and the polishing degree was monitored in real time by detecting the thickness of the residual silicon dioxide nanofilm during the polishing, such that the polishing process can be stable and controllable and feature higher uniformity. Meanwhile, the SiO₂ film may be used as a passivation protective layer of the substrate to ensure that the chip is stable in the surface modification and polishing process and in the use of the chip.

### Comparative Example 1

The procedures are the same as those in Example 4 except that the patterned glue layer was formed by directly nano-imprinting on the surface of the silicon chip substrate, without the use of the silicon dioxide film.

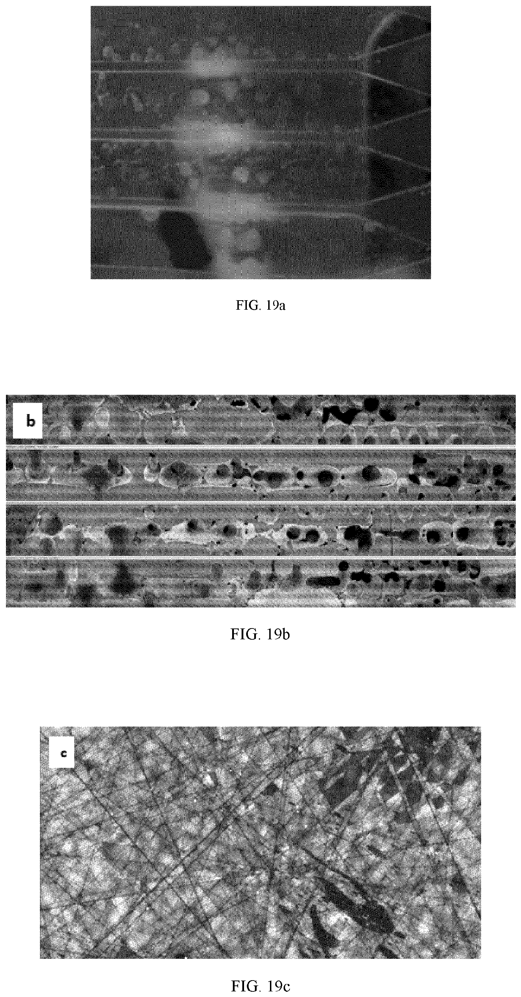

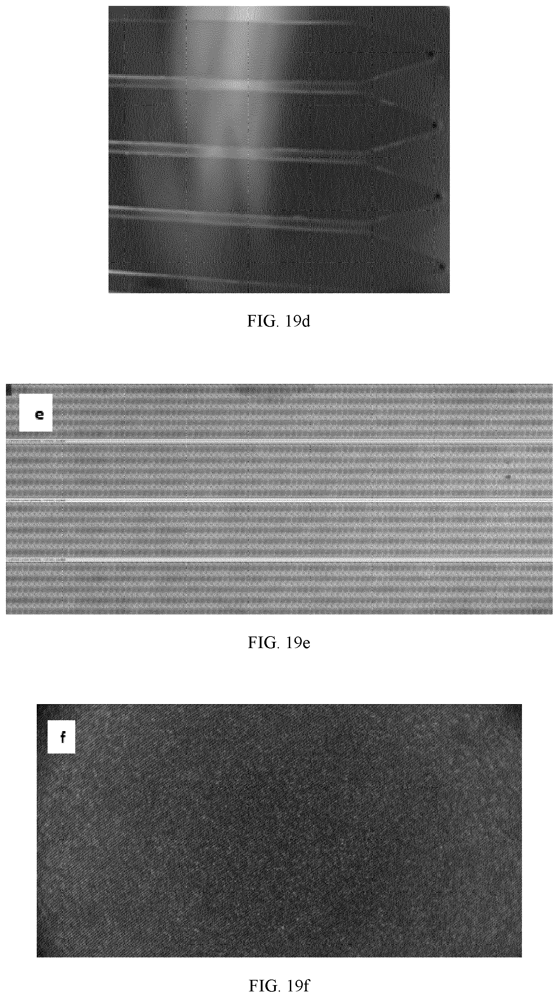

The surfaces of the biochips after polishing in Example 5 and Comparative Example 1 were detected, and the results are shown in FIGs. 19a-19f. FIG. 19a is a structural schematic of the biochip prepared in Comparative Example 1, FIG. 19b is a fluorescent image illustrating the multiple channels of the biochip prepared in Comparative Example 1, FIG. 19c is a partial surface fluorescent image of the biochip prepared in Comparative Example 1, FIG. 19d is a structural schematic of the biochip prepared in Example 4, FIG. 19e is a fluorescent image illustrating the multiple channels of the biochip prepared in Example 4, and FIG. 19f is a partial surface fluorescent image of the biochip prepared in Example 4.

As shown in FIGs. 19a-19f, the polishing of the biochip without SiO₂ film on the surface was not uniform, the surface pattern was destroyed after polishing, and the surface modification produced a large number of bubbles, such that the surface macromolecules and the subsequent modification with the detection molecules (nucleic acid probes, proteins, etc.) were not uniform and a large number of bubbles were observed (FIGs. 19a, 19b, and 19c). Through the deposition of the surface SiO₂ film, surface patterning, and subsequent surface modification and polishing, the surface polishing degree was well controlled, leading to uniform surface polishing and modification and stable surface processes (the modification with detection molecule and biomolecule detection).

### Example 6

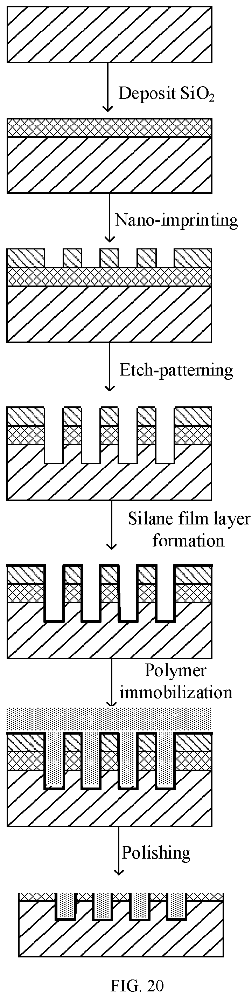

Now refer to FIG. 20 illustrating the method for preparing a biochip according to Example 6 of the present application:
Firstly, a silicon dioxide film with a thickness of 10 nm was formed on the surface of a silicon chip substrate by chemical vapor deposition; a patterned glue layer was then formed on the surface of the silicon dioxide film by nano-imprinting; the chip substrate was etched according to the pattern; regularly arranged cylindrical depressions were formed on the silicon chip substrate, where the cylindrical depression had a depth of 300 nm, a cross-sectional diameter of 500 nm, and a distance (from center to center) between two adjacent cylindrical depressions of 3 µm; the cylindrical depressions were arranged in a honeycomb pattern; that is, the cylindrical depressions were arranged in rows and columns, the cylindrical depressions of two adjacent rows were not located in the same column, and the cylindrical depressions of alternate rows were located in the same column; the cylindrical depressions of two adjacent columns were not located in the same row, and the cylindrical depressions of alternate columns were located in the same row.

After forming the patterned chip substrate, a silane molecule film layer containing an amino functional group was formed in the cylindrical depression, and a polymer was then immobilized on the chip substrate by amino-carboxyl condensation; the bio macromolecule layer, the small molecule layer, the glue layer, and part of the silicon dioxide film outside the cylindrical depression were removed by polishing the resultant chip substrate using a zirconium oxide polishing solution with the silicon dioxide film being the polishing endpoint; the polished chip substrate was cut, cleaned and encapsulated to give the biochip.

During the polishing, with the silicon dioxide film being the polishing endpoint, and the polishing degree was monitored in real time by detecting the thickness of the residual silicon dioxide nanofilm during the polishing, such that the polishing process can be stable and controllable and feature higher uniformity. Meanwhile, the SiO₂ film may be used as a passivation protective layer of the substrate to ensure that the chip is stable in the surface modification and polishing process and in the use of the chip.

### Comparative Example 2

The procedures are the same as those in Example 6 except that the patterned glue layer was formed by directly nano-imprinting on the surface of the silicon chip substrate, without the use of the silicon dioxide film.

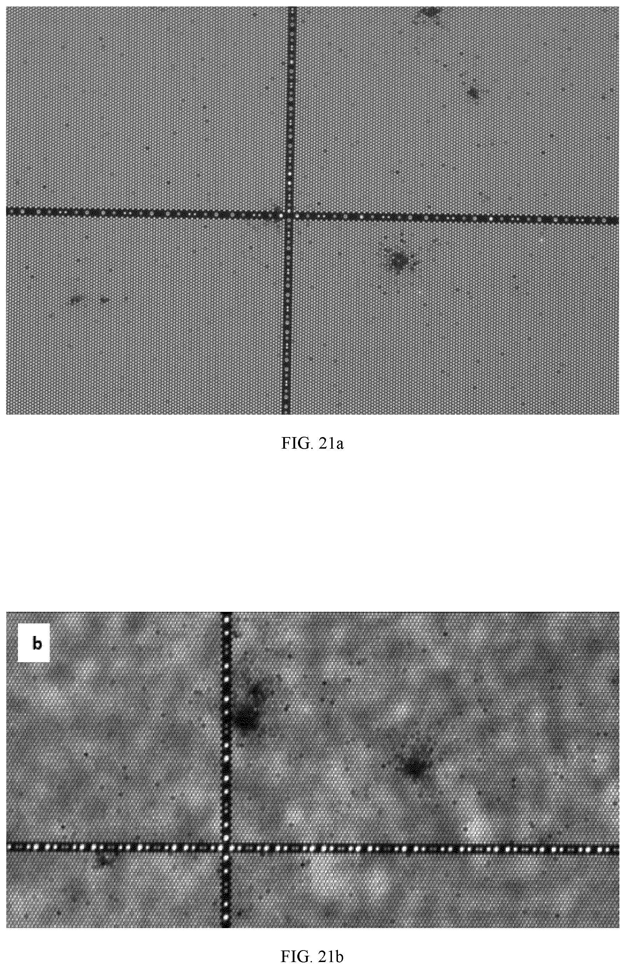

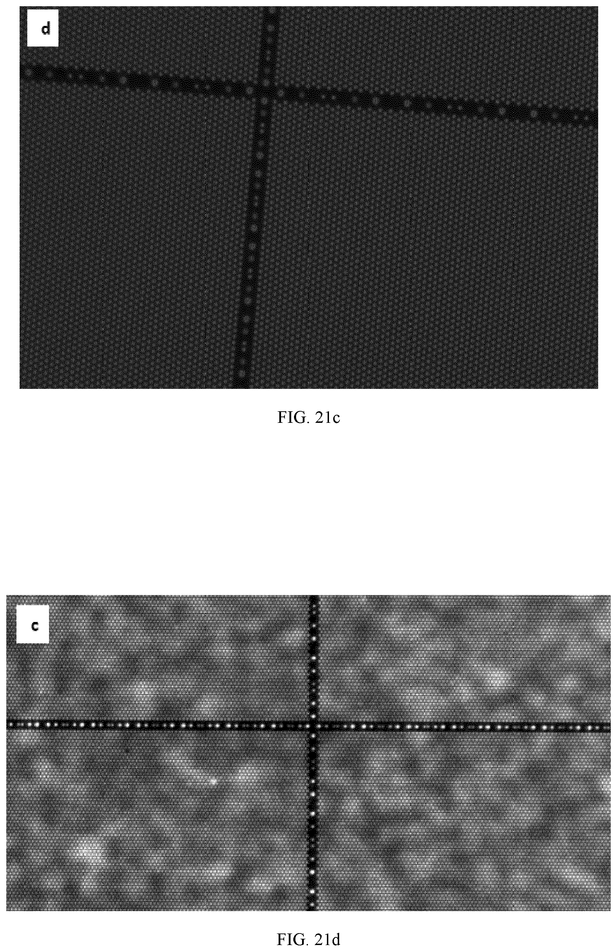

The surfaces of the biochips after polishing in Example 6 and Comparative Example 2 were detected, and the results are shown in FIGs. 21a-21d. FIG. 21a is an image illustrating the surface of the biochip prepared in Comparative Example 2, FIG. 21b is a partially enlarged view of FIG. 21a, FIG. 21c is an image illustrating the surface of the biochip prepared in Example 6, and FIG. 21d is an enlarged view illustrating the fluorescence on the surface of the biochip prepared in Example 6.

As can be seen from FIGs. 21a-21d, the use of the silicon dioxide film as the detection endpoint can avoid mechanical damage to the surface due to the difficulties in controlling the chemomechanical polishing process and maximize the integrity of the surface pattern.

### Example 7

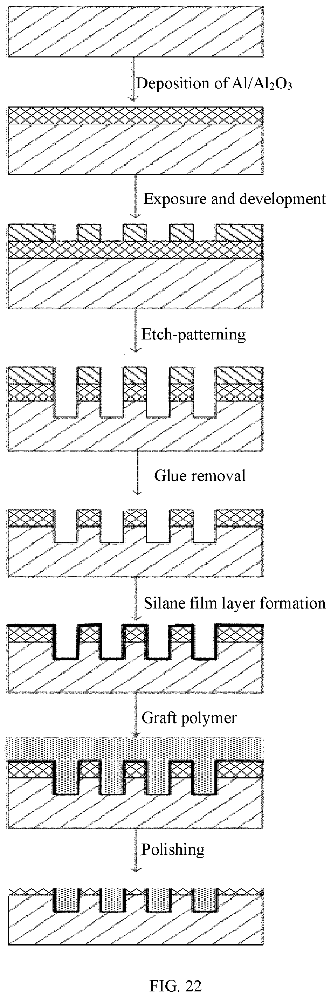

Now refer to FIG. 22 illustrating the method for preparing a biochip according to Example 7 of the present application:
An Al/Al₂O₃ film with a thickness of 50 nm was deposited on a quartz chip substrate wafer to give a chip substrate;
A photoresist was applied to the chip substrate, and etched after exposure and development; after removing the photoresist, geometric structures in a honeycomb arrangement were formed, where the geometric structure was a cylinder depression with a depth of 300 nm, a circular bottom surface diameter of 400 nm, and a distance (from center to center) between two adjacent cylindrical depressions of 800 nm; the cylindrical depressions were arranged in rows and columns, the cylindrical depressions of two adjacent rows were not located in the same column, and the cylindrical depressions of alternate rows were located in the same column; the cylindrical depressions of two adjacent columns were not located in the same row, and the cylindrical depressions of alternate columns were located in the same row.

A silane molecule film layer was formed on the geometric structure and reacted with a polymeric monomer solution to graft the polymer on the silane molecule. The chip was then polished and macromolecules outside the patterned area were removed to give the biochip.

During the polishing, with the Al/Al₂O₃ film being the polishing endpoint, and the polishing degree was monitored in real time by detecting the thickness of the residual Al/Al₂O₃ nanofilm during the polishing, such that the polishing process can be stable and controllable and feature higher uniformity.

### Comparative Example 3

The procedures are the same as those in Example 7 except that the photoresist was directly applied to the quartz chip substrate and then etched after exposure and development to form a patterned glue layer without using the Al/Al₂O₃ film.

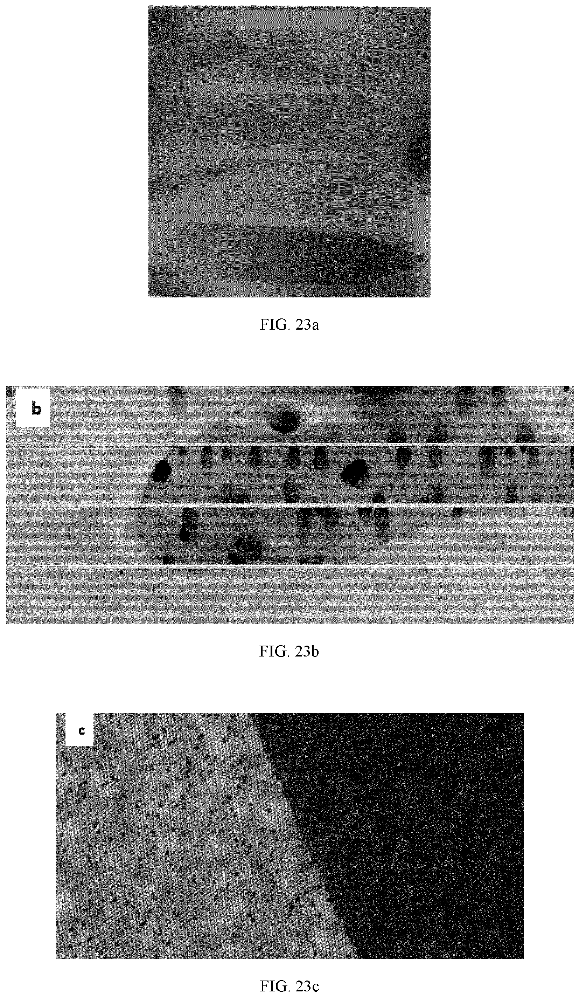

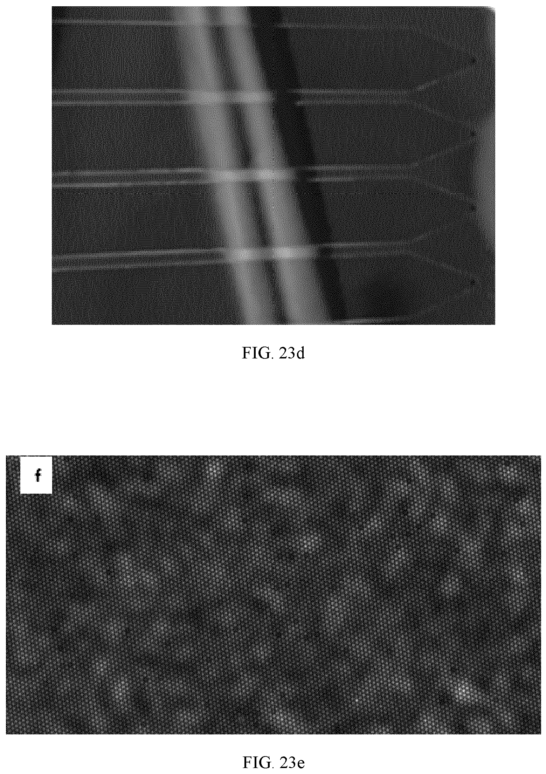

The surfaces of the biochips after polishing in Example 7 and Comparative Example 3 were detected, and the results are shown in FIGs. 23a-23e. FIG. 23a is an image illustrating the surface of the biochip prepared in Comparative Example 3, FIG. 23b is a fluorescent image illustrating the multiple channels of the biochip prepared in Comparative Example 3, FIG. 23c is an enlarged view illustrating the fluorescence on the surface of the biochip prepared in Comparative Example 3, FIG. 23d is an image illustrating the surface of the biochip prepared in Example 5, and FIG. 23e is an enlarged view illustrating the fluorescence on the surface of the biochip prepared in Example 5. As can be seen, the surface without an endpoint detection functionality (FIG. 23a) easily led to non-uniform polishing, and the chip with the endpoint detection functionality (FIG. 23d) resulted in a uniform surface. By staining the surface with a general fluorescent staining method and photographing, it can be seen that areas with non-uniform polishing led to uniform surface modification and a greater number of defects in the patterned areas due to excessive damage to the macromolecule modification by polishing (FIGs. 23b and 23c), while the polishing results were more uniform with fewer defects in the patterned areas for the chip with detectable polishing endpoints (FIG. 23e). The polishing endpoint detection achieved by the surface nanofilm can give a more stable and uniform chip surface.

### Example 8

Firstly, a silicon dioxide film was formed on the surface of a silicon chip substrate by physical vapor deposition; a photoresist was then applied to the surface of the silicon dioxide film; after exposing, developing, and etching the photoresist, regularly arranged cylindrical depressions were formed on the silicon chip substrate, where the cylindrical depression had a depth of 300 nm, a cross-sectional diameter of 300 nm, and a distance (from center to center) between two adjacent cylindrical depressions of 1 µm; the cylindrical depressions were arranged in a honeycomb pattern; that is, the cylindrical depressions were arranged in rows and columns, the cylindrical depressions of two adjacent rows were not located in the same column, and the cylindrical depressions of alternate rows were located in the same column; the cylindrical depressions of two adjacent columns were not located in the same row, and the cylindrical depressions of alternate columns were located in the same row.

After forming the patterned chip substrate, a silane molecule layer containing an amino functional group in the cylindrical depression, and a polymer was then immobilized on the chip substrate by amino-carboxyl condensation; the bio macromolecule layer, the small molecule layer, and the aluminum film outside the cylindrical depression were removed by polishing the resultant chip substrate using a zirconium oxide polishing solution with the aluminum film being the polishing endpoint; the polished chip substrate was cut, cleaned and encapsulated to give the biochip.

The above description is only illustrative of the preferred embodiments of the present disclosure, and it should be noted that those of ordinary skill in the art can make, without departing from the principles of the present disclosure, various improvements and modifications, and such modifications and modifications shall also be construed within the scope of the present disclosure.

## Claims

1. A chip, comprising a chip substrate, wherein the chip substrate is provided with regularly arranged geometric structures on at least one surface;
the geometric structure has a height of 10 nm to 100 µm in a direction perpendicular to the surface of the chip and a maximum dimension of 10 nm to 100 µm in a direction parallel to the surface of the chip; the distance between adjacent geometric structures is 10 nm to 100 µm.

2. The chip according to claim 1, wherein the geometric structure has a height of 100 nm to 1 µm in a direction perpendicular to the surface of the chip and a maximum dimension of 100 nm to 1 µm in a direction parallel to the surface of the chip;
the distance between adjacent geometric structures is 100 nm to 1 µm.

3. The chip according to claim 1, wherein the geometric structure is a depression or protrusion formed on the surface of the chip substrate.

4. The chip according to claim 3, wherein the geometric structure is one or a combination of more of a cylinder, a cone, a conical frustum, a prism, a pyramid, a frustum, or an irregular shape.

5. The chip according to claim 1, wherein the surface is provided with at least one array unit having the regularly arranged geometric structures.

6. The chip according to claim 5, wherein the array unit is one or more of a square array unit, a rectangular array unit, and a honeycomb array unit.

7. The chip according to any one of claims 1 to 6, wherein the chip further comprises a first cover plate bonded to the surface of the chip substrate provided with the geometric structure, and the first cover plate is made of a material having a light transmittance of 95% or greater.

8. The chip according to claim 7, wherein the surface of the first cover plate facing the chip substrate is a flat surface; or
the surface of the first cover plate facing the chip substrate is provided with regularly arranged geometric structures, and the geometric structure of the first cover plate is identical to or different from that of the chip substrate.

9. The chip according to any one of claims 1 to 6, wherein the chip substrate is provided with regularly arranged geometric structures on both surfaces.

10. The chip according to any one of claims 1 to 6, wherein the chip comprises a first cover plate and a second cover plate respectively bonded to the two surfaces of the chip substrate, and the first cover plate and the second cover plate are made of a material having a light transmittance of 95% or greater.

11. The chip according to claim 10, wherein two surfaces of the first cover plate and the second cover plate are flat surfaces; or
the first cover plate is provided with a geometric structure on one surface, the geometric structure of the first cover plate is arranged opposite to the geometric structure of the chip substrate, and two surfaces of the second cover plate are flat surfaces;
or
the first cover plate and the second cover plate are both provided with a geometric structure on one surface, and the geometric structures on the first cover plate and the second cover plate are respectively opposite to the geometric structures on the two surfaces of the chip substrate.

12. The chip according to any one of claims 1 to 11, wherein the chip comprises a polymer layer arranged on the surface provided with the geometric structure.

13. The chip according to claim 12, wherein the polymer layer is formed from a polymer containing a first functional group selected from one or more of sulfydryl, formyl, hydroxyl, carboxyl, amino, alkenyl, alkynyl, and azido.

14. The chip according to claim 12, wherein the polymer layer is bonded to the surface of the chip substrate and/or the cover plate via a coupling molecule.

15. The chip according to claim 14, wherein the coupling molecule has the following formula:
XₐSi(Y_{b})M_{c}Z,
X is H, F, Cl, Br, I, C₁₋₆ alkyl, C₁₋₆ haloalkyl, or C₁₋₆ alkoxy;
Y is selected from C₁₋₁₀ alkyl;
M_{c} is selected from C₁₋₅₀ alkylene, -(OCH₂CH₂)_{c}-, and -(CH(OH)CH₂-)_{c};
Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl,
C₆₋₂₀ aryl, and 5- to 20-membered heteroaryl;
a, b, and c are integers meeting the following conditions: 0 ≤ a ≤ 3, 0 ≤ b ≤ 3, and a + b = 3; 1 ≤ c ≤ 50.

16. The chip according to any one of claims 12 to 15, further comprising a first film layer arranged between the chip substrate and/or the cover plate and the polymer layer, wherein the first film layer is made of a material different from that of the surface to which it is bonded.

17. The chip according to claim 16, wherein the first film layer is selected from one or more of a metal film, a metal oxide film, and an inorganic oxide film.

18. The chip according to claim 17, wherein the first film layer is made of at least one of Au, Al, and SiO₂.

19. The chip according to claim 16, wherein the first film layer has a thickness of 10 to 1000 nm.

20. The chip according to any one of claims 7 to 19, wherein a glue layer is arranged between the chip substrate and the cover plate, and the glue layer has a channel arranged in a length direction of the chip; the geometric structure is arranged on the surface of the chip substrate and/or the cover plate in a region where the channel is located, and the shapes and/or arrangements of the geometric structures in different channels on the same surface are identical or different.

21. A method for preparing a chip, comprising:
a) forming regularly arranged geometric structures on at least one surface of a substrate,
wherein the geometric structure has a height of 10 nm to 100 µm in a direction perpendicular to the surface of the substrate and a maximum dimension of 10 nm to 100 µm in a direction parallel to the surface of the substrate;
the distance between adjacent geometric structures is 10 nm to 100 µm.

22. The method according to claim 21, wherein the substrate is a chip substrate and/or a cover plate.

23. The method according to claim 21, further comprising:
b) forming a polymer layer on the geometric structure.

24. The method according to claim 23, wherein the geometric structure is a depression formed on the surface of the chip substrate and/or the cover plate, and b) comprises:
bonding a polymer containing a first functional group to the surface provided with the geometric structure; and
polishing the polymer to remove the polymer outside the region where the geometric structure is located, so as to form the polymer layer on the geometric structure.

25. The method according to claim 24, wherein the polishing is chemical polishing and/or mechanical polishing.

26. The method according to any one of claims 21 to 25, wherein the geometric structure is a protrusion formed on the surface of the chip substrate and/or the cover plate, and the method comprises, before a):
bonding the polymer containing the first functional group to the surface of the substrate.

27. The method according to claim 26, wherein the first functional group is selected from one or more of sulfydryl, formyl, hydroxyl, carboxyl, amino, alkenyl, alkynyl, and azido.

28. The method according to claim 26, further comprising, before bonding the polymer: processing the substrate with a coupling molecule.

29. The method according to claim 28, wherein the coupling molecule has the following formula:
XₐSi(Y_{b})M_{c}Z,
X is H, F, Cl, Br, I, C₁₋₆ alkyl, C₁₋₆ haloalkyl, or C₁₋₆ alkoxy;
Y is selected from C₁₋₁₀ alkyl;
M_{c} is selected from C₁₋₅₀ alkylene, -(OCH₂CH₂)_{c}-, and -(CH(OH)CH₂-)_{c};
Z is selected from the following unsubstituted or substituted groups: amino, carboxyl, hydroxyl, azido, C₁₋₂₀ alkyl, C₂₋₂₀ alkenyl, C₂₋₂₀ alkynyl, C₃₋₂₀ cycloalkyl, 3- to 20-membered heterocyclyl, C₆₋₂₀ aryl, and 5- to 20-membered heteroaryl;
a, b, and c are integers meeting the following conditions: 0 ≤ a ≤ 3, 0 ≤ b ≤ 3, and a + b = 3; 1 ≤ c ≤ 50.

30. The method according to any one of claims 21 to 29, further comprising: before a), or after a) and before b), preparing a first film layer on the surface provided with the geometric structure, wherein the first film layer and the substrate are made of different materials.

31. The method according to claim 30, wherein the first film layer is selected from one or more of a metal film, a metal oxide film, and an inorganic oxide film.

32. The method according to claim 31, wherein the first film layer is made of at least one of Au, Al, and SiO₂.

33. The method according to claim 31, wherein the first film layer has a thickness of 10 to 1000 nm.

34. The method according to any one of claims 21 to 33, wherein optionally, the substrate comprises at least a chip substrate, and the method further comprises:
bonding the cover plate to the surface of the chip substrate provided with the geometric structure using an adhesive material, and forming a channel in the adhesive material in a length direction of the chip.

35. The method according to any one of claims 21 to 34, wherein the chip is the chip according to any one of claims 1 to 20.
